# EUROPÄISCHE PATENTSCHRIFT

(11) **EP 1 763 300 B1**
(45) Veröffentlichungstag und Bekanntmachung des Hinweises auf die Patenterteilung: **15.05.2013**
(21) Anmeldenummer: 05761142.8
(22) Anmeldetag: 28.06.2005
(51) Int. Cl.: A01N 25/30, A01N 25/02, A01N 47/24, A61K 8/04, A61K 8/81, A61K 9/14, A61K 47/34, A61Q 19/00, C08F 26/02, C08F 28/02, C08F 20/42, C08F 22/30

(54) **VERWENDUNG VON ETHERGRUPPEN ENTHALTENDEN POLYMEREN ALS LÖSUNGSVERMITTLER**
USE OF ETHER GROUP-CONTAINING POLYMERS AS SOLUBILIZERS
UTILISATION DE POLYMERES CONTENANT DES GROUPES ETHER EN TANT QU'AGENTS DE SOLUBILISATION

(30) Priorität: 28.06.2004 DE 102004031158
(43) Veröffentlichungstag der Anmeldung: 21.03.2007
(73) Patentinhaber: BASF SE, 67056 Ludwigshafen (DE)
(72) Erfinder: PIEROBON, Marianna, 67063 Ludwigshafen (DE); BOUILLO, Nathalie, 76532 Baden-Baden (DE); KLÜGLEIN, Matthias, 67071 Ludwigshafen (DE); LANGE, Ronald Frans Maria, 67061 Ludwigshafen (DE); KÖHLE, Harald, 67273 Bobenheim (DE); SCHERER, Maria, 76829 Godramstein (DE); KRÜGER, Christian, 55291 Saulheim (DE); KOLTZENBURG, Sebastian, 67125 Dannstadt-Schauernheim (DE); BRATZ, Matthias, 67133 Maxdorf (DE); OETTER, Günter, 67227 Frankenthal (DE); ZUAZO, Izaskun Manteca DI, decease (DE)
(86) Internationale Anmeldenummer: PCT/EP2005/053123
(87) Internationale Veröffentlichungsnummer: WO 2006/000592

(56) Entgegenhaltungen:
- WO-A-97/43324
- WO-A-2005/018321
- DD-A- 75 402
- US-A- 5 770 555

## Beschreibung

Diese Anmeldung nimmt die Priorität der DE 10 2004 031 158.7 in Anspruch.

Die vorliegende Erfindung betrifft die Verwendung von Ethergruppen enthaltenden Polymeren als Lösungsvermittler, insbesondere bei Kosmetika, Arzneimitteln, Nahrungsmitteln, Nahrungsergänzungsmitteln und Futtermitteln, ganz besonders bevorzugt bei der Herstellung von Pflanzenschutzmitteln, wobei die Polymere erhältlich sind durch Copolymerisation von
(A) (Meth)acrylsäure oder Maleinsäureanhydrid,
(B) Styrol,
(C) mindestens eines alkoxylierten ungesättigten Ethers der allgemeinen Formel I 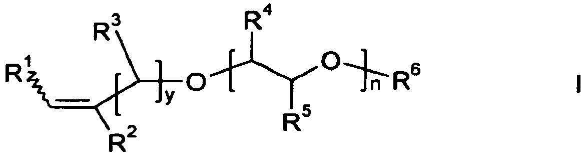
(D) optional weiteren monoethylenisch ungesättigten Monomeren,
   wobei in Formel I die Variablen wie folgt definiert sind:
   - R¹ und R³: Wasserstoff;
   - R²: gewählt aus Wasserstoff oder Methyl;
   - R⁴, R⁵: jeweils Wasserstoff;
   - R⁶: gewählt aus Wasserstoff oder C₁-C₄-Alkyl,
   - n: eine ganze Zahl von 3 bis 100,
   - y: 1.

Aus dem Stand der Technik sind die unterschiedlichsten Polymere für die verschiedensten Anwendungen bekannt.

In EP 0 628 085 wird die Verwendung von Copolymeren aus Maleinsäureanhydrid und optional einem zweiten Monomer, beispielsweise Styrol, Isobuten oder Vinylacetat beschrieben, wobei die Copolymeren vor der Verwendung zum Nachgerben und Füllen mit alkoxylierten Alkoholen umgesetzt werden.

Aus EP 0 792 377 ist ein Verfahren bekannt, bei dem die Gerbung und gegebenenfalls auch die Vorgerbung durch Aldehyde oder andere reaktive Carbonylverbindungen in Anwesenheit von Polymeren, beispielsweise Maleinsäureanhydrid-alpha-Olefin-Styrol-Terpolymeren (Variante I), durchgeführt wird.

Aus US 5,646,225 und US 5,728,777 sind Copolymere aus Styrol, Methacrylsäure und mono- *und* doropoxyliertem Allylalkohol sowie die Verwendung der Copolymere in Tinten auf *wäßriger und* Lösungsmittelbasis (US 5,728,777) bekannt.

Die US 4,847,410 und US 4,959,156 beschreiben die Herstellung von Copolymeren aus Allylalkoholalkoxylaten und (Meth)acrylsäure und deren Einsatz als Dispergiermittel.

In der EP 0 601 536 werden ferner wasserlösliche Pfropfcopolymere beschrieben, die als Dispergiermittel in wäßrigen anorganischen Bindemitteln verwendbar sind.

Aus WO 01/96007 sind wasserlösliche Copolymere bekannt, die durch Copolymerisation von (Meth)acrylsäure oder von Maleinsäurehalbestern, einem alkylverkappten alkoxylierten Halbester und optional einer Dicarbonsäure sowie optional Styrol hergestellt werden, und ihre Verwendung als Dispergiermittel für Pigmente wie beispielsweise CaCO3 ist beschrieben.

Darüber hinaus gibt es im Bereich der Wirkstoffe einschließlich des Pflanzenschutzbereichs zahlreiche Polymere mit den verschiedensten Anwendungsmöglichkeiten. Beispielhaft sei auf die WO 03/043420 verwiesen. Sie beschreibt die Verwendung von Copolymeren als Adjuvantien bei der Behandlung von Pflanzen. Die Copolymere bestehen aus Olefinen und/oder Vinylethern, sowie aus ethylenisch ungesättigten Dicarbonsäuren bzw. Dicarbonsäurederivaten und weiteren Comonomeren.

Hinzuweisen ist auch auf die DE 103 38 437, die die Formulierung von Agrowirkstoffen mit Adjuvantien auf der Basis von Blockcopolymeren beschreibt.

Ebenso beschreibt die DE 103 51 004 statistische radikalische Polymerisate auf Basis von sulfonathaltigen Acrylaten und ihre Verwendung zur Wirkstofformulierung insbesondere im Pflanzenschutzbereich.

Aus der EP 002820951 sind ferner Depotpräparate für Wirkstoffe bekannt, die aus einem Copolymer bestehend aus Allylalkoholalkoxylaten und Maleinsäureanhydrid aufgebaut sind.

Aus der US 5,770,555 sind Reinigungszusammensetzungen bekannt, die polyolbasierte Polymere umfassen

Abschließend sei noch darauf hingewiesen, daß aus der US 2,872,369 Copolymere bekannt sind, welche die Löslichkeit von schwer löslichen Komponenten in polaren Lösemitteln erhöhen.

Ziel der vorliegenden Erfindung ist es nunmehr, hydrophobe, wasserlösliche Wirk- und Effektstoffe mit Hilfe von Polymeren in eine stabile wäßrige Formulierung zu überführen.

Stabil bedeutet im Sinne der Erfindung, daß die Formulierungen sedimentations- und aufrahmstabil über einen für die jeweilige Anwendung hinreichenden Zeitraum und Temperaturbereich sind. Unter Aufrahmen (creaming) versteht man dabei die Anreicherung der meist spezifisch leichteren, dispergierten Öltropfen im oberen Teil einer Öl-in-Wasser Emulsion. So ist beispielsweise für eine Anwendung im Pflanzenschutz die Stabilität einer Wirkstoff-Formulierung über ein oder mehrere Jahre Voraussetzung. Zur Erhöhung der Wirkung und der Bioverfügbarkeit von gering wasserlöslichen Wirk- und Effektstoffen können diese mit Hilfe der Polymeren im Extremfall solubilisiert sein.

Als ein Beispiel sei die Solubilisierung von Wirkstoffen in tensidartigen Polymermizellen genannt, wodurch die Wirkstoffe molekulardispers in der wäßrigen Formulierung stabilisiert sind. Molekulardispers heißt in diesem Zusammenhang, daß keine Wirkstoffkristalle im Transmissionselektronenmikroskop nachgewiesen werden können.

Aufgabe der vorliegenden Erfindung war es daher, Polymere zur Verfügung zu stellen, welche als Lösungsvermittler insbesondere bei Kosmetika, Arzneimitteln, Nahrungsmitteln, Nahrungsergänzungsmitteln und Futtermitteln, ganz besonders bei der Herstellung von Pflanzenschutzmitteln einsetzbar sind.

Die erfindungsgemäße Aufgabe wird gelöst durch die Verwendung von Ethergruppen enthaltenden Polymeren als Lösungsvermittler, insbesondere bei Kosmetika, Arzneimitteln, Nahrungsmitteln, Nahrungsergänzungsmitteln und Futtermitteln, ganz besonders bei der Herstellung von Pflanzenschutzmitteln wobei die Polymere erhältlich sind durch Copolymerisation von
(A) 5 bis 30 Gew.-% (Meth)acrylsäure oder Maleinsäureanhydrid, bevorzugt (Meth)Acrylsäure,
(B) 5 bis 80 Gew.-% Styrol,
(C) 10 bis 90 Gew.-% mindestens eines alkoxylierten ungesättigten Ethers der allgemeinen Formel I 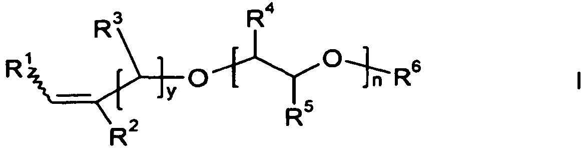
(D) 0 bis 20 Gew.-% optional weiteren monoethylenisch ungesättigten Monomeren,
   wobei in Formel I die Variablen wie folgt definiert sind:
   - R¹ und R³: Wasserstoff;
   - R²: gewählt aus Wasserstoff oder Methyl;
   - R⁴, R⁵: jeweils Wasserstoff;
   - R⁶: gewählt aus Wasserstoff oder C₁-C₄-Alkyl, bevorzugt Wasserstoff,
   - n: eine ganze Zahl von 3 bis 100, bevorzugt 10 bis 40, insbesondere 3 bis 40
   - y: 1.

Bei dem Begriff Lösungsvermittler handelt es sich um die Bezeichnung für häufig grenzflächenaktive Stoffe, die durch ihre Gegenwart andere, in einem bestimmten Lösungsmittel praktisch unlösliche Verbindungen in diesem Lösungsmittel löslich oder emulgierbar machen (Solubilisation). Es gibt Lösungsvermittler, die mit der schwer löslichen Substanz eine Molekülverbindung eingehen und solche, die durch Micell-Bildung wirken bzw. auch solche, die einem sogenannten latenten Lösemittel sein Lösungsvermögen verleihen.

Derartige Polymere sind bereits aus der DE 103 33 749 bekannt. In dieser Anmeldung wird jedoch nur die Verwendung für die Lederbearbeitung beschrieben. Weitere Verwendungsmöglichkeiten sind nicht erwähnt.

Erfindungsgemäß ist es nunmehr überraschend, daß die beschriebenen Copolymere sich auch als Lösungsvermittler, insbesondere bei Kosmetika, Arzneimitteln, Nahrungsmitteln, Nahrungsergänzungsmitteln und Futtermitteln, ganz besonders bei der Herstellung von Pflanzenschutzmitteln eignen.

Der Gehalt an Wirk- und/oder Effektstoff kann über weite Bereiche variiert werden. Insbesondere ermöglichen die amphiphilen Polymerzusammensetzungen das Herstellen von so genannten Wirkstoffkonzentraten, welche den Wirkstoff in einer Menge von wenigstens 5 Gew.-%, z. B. in einer Menge von 5 bis 50 Gew.-% und insbesondere in einer Menge von 5 bis 20 Gew.-%, bezogen auf das Gesamtgewicht der Zusammensetzung, enthalten.

Vorteilhafterweise können die erfindungsgemäßen wäßrigen Wirkstoffzusammensetzungen lösungsmittelfrei oder lösungsmittelarm formuliert werden, d. h. der Anteil an organischen Lösemitteln in der wäßrigen Wirkstoffzusammensetzung beträgt häufig nicht mehr als 10 Gew.-%, insbesondere nicht mehr als 5 Gew.-% und insbesondere nicht mehr als 1 Gew.-%, bezogen auf das Gesamtgewicht der Zusammensetzung.

In den erfindungsgemäßen wäßrigen Zusammensetzungen können eine Vielzahl unterschiedlicher Wirk- und Effektstoffe formuliert werden. Eine besondere Ausführungsform der Erfindung betrifft die Formulierung von Wirkstoffen für den Pflanzenschutz, d.h. von Herbiziden, Fungiziden, Nematiziden, Akariziden, Insektiziden sowie Wirkstoffen, die das Pflanzenwachstum regulieren.

Beispiele für fungizide Wirkstoffe, die als erfindungsgemäße wässrige Wirkstoffzusammensetzung formuliert werden können, umfassen:

Pestizide sind dem Fachmann aus der Literatur bekannt. Der Begriff "Pestizid" bedeutet hier mindestens ein Wirkstoff ausgewählt aus der Gruppe der Insektizide, Fungizide, Herbizide und/oder Safener, Wachstumsregulatoren (s. Pesticide Manual, 13th Ed. (2003),

### Insektizide

- Organo(thio)phosphate wie Acephate, Azamethiphos, Azinphos-methyl, Chlorpyrifos, Chlorpyriphos-methyl, Chlorfenvinphos, Diazinon, Dichlorphos, Dicrotophos, Dimethoate, Disulfoton, Ethion, EPN, Fenitrothion, Fenthion, Isoxathion, Malathion, Methamidophos, Methidathion, Methyl-Parathion, Mevinphos, Monocrotophos, Oxydemeton-methyl, Paraoxon, Parathion, Phenthoate, Phosalone, Phosmet, Phosphamidon, Phorate, Phoxim, Pirimiphosmethyl, Profenofos, Prothiofos, Primiphos-ethyl, Pyraclofos, Pyridaphenthion, Sulprophos, Triazophos, Trichlorfon, Tetrachlorvinphos, Vamidothion
- Carbamate wie Alanycarb, Benfuracarb, Bendiocarb, Carbaryl, Carbofuran Carbosulfan, Fenoxycarb, Furathiocarb, Indoxacarb, Methiocarb, Methomyl, Oxamyl, Pirimicarb, Propoxur, Thiodicarb, Triazamate;
- Pyrethroide wie Allethrin, Bifenthrin, Cyfluthrin, Cycloprothrin, Cyphenothrin, Cypermethrin sowie die alpha-, beta-, theta- und zeta-Isomere, Deltamethrin, Esfenvalerate, Ethofenprox, Fenpropathrin, Fenvalerate, Cyhalothrin, Lambda-Cyhalothrin,
   Imiprothrin, Permethrin, Prallethrin, Pyrethrin I, Pyrethrin II, Silafluofen, Tau-Fluvalinate, Tefluthrin, Tetramethrin, Tralomethrin, Alpha-Cypermethrin Transfluthrin, Zeta-Cypermethrin;
- Arthropode Wachstumsregulatoren wie a) Chitinsyntheseinhibitoren; z. B. Benzoylharnstoffe wie Chlorfluazuron, Cyromacin, Diflubenzuron, Flucycloxuron, Flufenoxuron, Hexaflumuron, Lufenuron, Novaluron, Teflubenzuron, Triflumuron; Buprofezin, Diofenolan, Hexythiazox, Etoxazole, Clofentazine; b) Ecdysone Antagonisten wie Halofenozide, Methoxyfenozide, Tebufenozide; c) Juvenoide wie Pyriproxyfen, Methoprene, Fenoxycarb; d) Lipidbiosynthese-Inhibitoren wie Spirodiclofen;
- Neonicothinoide wie Flonicamid, Clothianidin, Dinotefuran, Imidacloprid, Thiamethoxam, Nitenpyram, Nithiazin, Acetamiprid, Thiacloprid;
- Pyrazol-Insektizide wie Acetoprole, Ethiprole, Fipronil, Tebufenpyrad, Tolfenpyrad und Vaniliprole;
- Weitere unklassifitierte Insektizide wie Abamectin, Acequinocyl, Acetamipirid, Amitraz, Azadirachtin, Bensutap, Bifenazate, Cartap, Chlorfenapyr, Chlordimeform, Cyromazine, Diafenthiuron, Dinetofuran, Diofenolan, Emamectin, Endosulfan, Ethiprole, Fenazaquin, Fipronil, Formetanate, Formetanate-Hydrochlorid, gamma-HCH, Hydramethylnon, Imidacloprid, Indoxacarb, Isoprocarb, Metolcarb, Piperonylbutoxid, Pyridaben, Pymetrozine, Spinosad, Tebufenpyrad, Thiamethoxam, Thiocyclam, XMC und Xylylcarb, Pyridalyl, Pyridalyl, Flonicamid, Fluacypyrim, Milbemectin, Spiromesifen, Flupyrazofos, NC 512, Tolfenpyrad, Flubendiamide, Bistrifluron, Benclothiaz, Pyrafluprole, Pyriprole, Amidoflumet, Flufenerim, Cyflumetofen, Acequinocyl, Lepimectin, Profluthrin, Dimefluthrin, Metaflumizone, N-R1-2,2-dihalo-1-R2-cyclo-propancarboxamid-2-(2,6-dichlor- a, a, a -tri-fluoro-p-tolyl)hydrazon, N-R1-2,2-di-R3-propionamid-2-(2,6-dichloro-a,a,atrifluoro-p-tolyl)-hydrazon,
   wobei R1 Methyl oder Ethyl, Halo Chlor oder Brom, R2 H (Wasserstoff) oder Methyl und R3 Methyl oder Ethyl ist,
   bevorzugt N-Ethyl-2,2-dichlor-1-methylcyclo-propanecarboxamid-2-(2,6-dichlor- a, a, a -tri-fluoro-p-tolyl)hydrazon, N-ethyl-2,2-dimethylpropionamide-2-(2,6-dichloro-a,a,atrifluoro-p-tolyl)-hydrazon, Säure der folgenden Formel

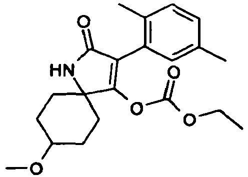

### Aminoiso-thiazol der Formel

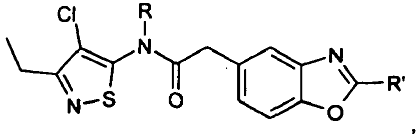

worin
R = -CH₂O CH₃ oder H und
R = -CF₂CF₂ CF₃;

### Anthranilamid der Formel

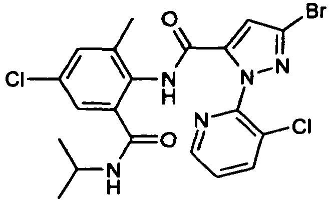

und eine insektizidaktive Verbindung der folgenden Formel

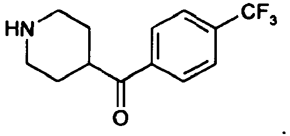

Die folgende Liste von Fungiziden zeigt mögliche Wirkstoffe auf, soll aber nicht auf diese beschränkt sein:
- Acylalanine z.B. Benalaxyl, Furalaxyl, Metalaxyl, Ofurace, Oxadixyl,
- Amin Derivate z.B. Aldimorph, Dodine, Dodemorph, Fenpropimorph, Fenpropidin, Guazatine, Iminoctadine, Spiroxamin, Tridemorph
- Anilinopyrimidine z.B. Pyrimethanil, Mepanipyrim oder Cyrodinyl,
- Antibiotika z.B. Cycloheximid, Griseofulvin, Kasugamycin, Natamycin, Polyoxin oder Streptomycin, Validamycin A
- Azole z.B. Bitertanol, Bromuconazole, Cyazofamide, Cyproconazole, Difenoconazole, Dinitroconazole, Epoxiconazole, Etridazole, Fenbuconazole,

Fluquiconazole, Flusilazole, Flutriafol, Fuberidazole, Hexaconazole, Hymexazole, Imazalil, lmibenconazole, Metconazole, Myclobutanil, Penconazole, Perfurazoate, Propiconazole, Prochloraz, Prothioconazole, Simeconazole, Tebuconazole, Tetraconazole, Thiabendazole Triadimefon, Triadimenol, Triflumizol, Triticonazole, 5-Chloro-7-(4-methyl-piperidin-1-yl)-6-(2,4,6-trifluoro-phenyl)-[1,2,4]Triazolo[1,5-a]Pyrimidine, 2-Butoxy-6-iodo-3-propyl-chromen-4-one, 3-(3-Bromo-6-fluoro-2-methyl-indole-1-sulfonyl)-[1,2,4]triazole-1-sulfonic acid dimethylamide,
- Dicarboximides z.B. Iprodion, Myclozolin, Procymidon, Vinclozol0in,
- Dithiocarbamates z.B. Ferbam, Nabam, Maneb, Mancozeb, Metam, Metiram, Propineb, Polycarbamate, Thiram, Ziram, Zineb,
- Heterocyclische Verbindungen wie Anilazine, Benomyl, Boscalid, Carbendazim, Carboxin, Oxycarboxin, Cyazofamid, Dazomet, Dithianon, Ethirimol, Dimethirimol, Famoxadon, Fenamidon, Fenarimol, Fuberidazole, Flutolanil, Furametpyr, Isoprothiolane, Mepronil, Nuarimol, Octhilinone, Picobezamid, Probenazole, Proquinazid, Pyrifenox, Pyroquilon, Quinoxyfen, Silthiofam, Thiabendazole, Thifluzamid, Thiophanate-methyl, Tiadinil, Tricyclazole, Triforine, 3-[5-(4-Chlorophenyl)-2,3-Dimethyl-Isoxazolidin-3-yl]-pyridine, Bupirimate
- Kupfer Fungizide z.B. Bordeaux mixture, Kufperacetat, Kufperhydroxid, Kufperoxychlorid, basisches Kupfersulfat,
- Nitrophenyl Derivatives z.B. Binapacryl, Dinocap, Dinobuton, Nitrophthalisopropyl
- Phenylpyrroles z.B. Fenpiclonil oder Fludioxonil,
- Schwefel
- Organische Metallverbindungen z.B. Fentin salze
- Organophosphorous Compounds z.B. Edifenphos, Edifenphos, Iprobenfos, Pyrazophos, Tolclofos-methyl, Fosetyl, Fosetyl-aluminium, Phosphorous Acid
- Andere Fungizide z.B. Acibenzolar-S-methyl, Benthiavalicarb, Carpropamid, Chlorothatonil, Cyflufenamid, Cymoxanil, Dazomet, Diclomezin, Diclocymet, Diethofencarb, Edifenphos, Ethaboxam, Fenhexamid, Fentin-acetate, Fenoxanil, Ferimzone, Fluazinam, Fosetyl, Fosetyl-Aluminum, Iprovalicarb,

Hexachlorobenzene, Metrafenon, Pencycuron, Propamocarb, Phthalide, Toloclofos-Methyl, Quintozene, Zoxamid, Isoprothiolane, Probenfos, Zoxamide, Fluopicolide (Picobenzamid); Carpropamid, Mandipropamid, N-(2-{4-[3-(4-Chlorophenyl)-prop-2-ynyloxy]-3-methoxy-phenyl}-ethyl)-2-methanesulfonylamino-3-methyl-butyramide, N-(2-{4-[3-(4-Chloro-phenyl)-prop-2-ynyloxy]-3-methoxyphenyl)-ethyl)-2-ethanesulfonylamino-3-methyl-butyramide; Furametpyr, Thifluzamide, Penthiopyrad, Fenhexamide, 3,4-Dichloro-isothiazole-5-carboxylic acid (2-cyano-phenyl)-amide, Flubenthiavalicarb, 3-(4-Chloro-phenyl)-3-(2-isopropoxycarbonylamino-3-methyl-butyrylamino)-propionic acid methyl ester, {2-Chloro-5-[1-(6-methyl-pyridin-2-ylmethoxyimino)-ethyl]-benzyl}-carbamic acid methyl ester, {2-Chloro-5-[1-(3-methyl-benzyloxyimino)-ethyl]-benzyl}-carbamic acid methyl ester, Fflusulfamide, Phthalide, Hexachlorbenzene

### Amide der Formel

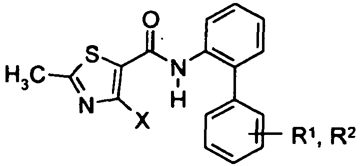

worin
X für CHF₂ oder CH₃; und
R¹,R² unabhängig voneinander für Halogen, Methyl oder Halomethyl; stehen
- Strobilurins z.B. Azoxystrobin, Dimoxystrobin, Enestroburin, Fluoxastrobin, Kresoxim-Methyl, Metominostrobin, Orysastrobin, Picoxystrobin, Pyraclostrobin oder Trifloxystrobin,
- Sulfenic Acid Derivate z.B. Captafol, Captan, Dichlofluanid, Folpet, Tolylfluanid,
- Cinnemamides und Analoga z.B. Dimethomorph, Flumetover or Flumorph,

Amide Fungicides z.B. Cyclofenamid or (Z)-N-[α-(cyclopropylmethoxyimino)-2,3-difluoro-6-(difluoromethoxy)benzyl]-2-phenylacetamide;

Die folgende Liste von Herbizden zeigt mögliche Wirkstoffe auf, soll aber nicht auf diese beschränkt sein:

Verbindungen, die die Biosynthese von Lipiden inhibieren, z.B. Chlorazifop, Clodinafop, Clofop, Cyhalofop, Ciclofop, Fenoxaprop, Fenoxaprop-p, Fenthiaprop, Fluazifop, Fluazifop-P, Haloxyfop, Haloxyfop-P, Isoxapyrifop, Metamifop, Propaquizafop, Quizalofop, Quizalofop-P, Trifop, Alloxydim, Butroxydim, Clethodim, Cloproxydim, Cycloxydim, Profoxydim, Sethoxydim, Tepraloxydim, Tralkoxydim, Butylate, Cycloat, Diallat, Dimepiperat, EPTC, Esprocarb, Ethiolate, Isopolinate, Methiobencarb, Molinate, Orbencarb, Pebulate, Prosulfocarb, Sulfallat, Thiobencarb, Thiocarbazil, Triallat, Vernolat, Benfuresat, Ethofumesat und Bensulid;

ALS-Inhibitoren wie Amidosulfuron, Azimsulfuron, Bensulfuron, Chlorimuron, Chlorsulfuron, Cinosulfuron, Cyclosulfamuron, Ethametsulfuron, Ethoxysulfuron, Flazasulfuron, Flupyrsulfuron, Foramsulfuron, Halosulfuron, Imazosulfuron, lodosulfuron, Mesosulfuron, Metsulfuron, Nicosulfuron, Oxasulfuron, Primisulfuron, Prosulfuron, Pyrazosulfuron, Rimsulfuron, Sulfometuron, Sulfosulfuron, Thifensulfuron, Triasulfuron, Tribenuron, Trifloxysulfuron, Triflusulfuron, Tritosulfuron, Imazamethabenz, Imazamox, Imazapic, Imazapyr, Imazaquin, Imazethapyr, Cloransulam, Diclosulam, Florasulam, Flumetsulam, Metosulam, Penoxsulam, Bispyribac, Pyriminobac, Propoxycarbazone, Flucarbazone, Pyribenzoxim, Pyriftalid und Pyrithiobac;

Verbindungen, die die Photosynthese inhibieren wie Atraton, Atrazine, Ametryne, Aziprotryne, Cyanazine, Cyanatryn, Chlorazine, Cyprazine, Desmetryne, Dimethametryne, Dipropetryn, Eglinazine, Ipazine, Mesoprazine, Methometon, Methoprotryne, Procyazine, Proglinazine, Prometon, Prometryne, Propazine, Sebuthylazine, Secbumeton, Simazine, Simeton, Simetryne, Terbumeton, Terbuthylazine und Terbutryne;

Protoporphyrinogen-IX Oxidase-Inhibitoren wie Acifluorfen, Bifenox, Cchlomethoxyfen, Chlomitrofen, Ethoxyfen, Fluorodifen, Fluoroglycofen, Fluoronitrofen, Fomesafen, Furyloxyfen, Halosafen, Lactofen, Nitrofen, Nitrofluorfen, Oxyfluorfen, Fluazolate, Pyraflufen, Cinidon-ethyl, Flumiclorac, Flumioxazin, Flumipropyn, Fluthiacet, Thidiazimin, Oxadiazon, Oxadiargyl, Azafenidin, Carfentrazone, Sulfentrazone, Pentoxazone, Benzfendizone, Butafenacil, Pyraclonil, Profluazol, Flufenpyr, Flupropacil, Nipyraclofen und Etnipromid;

Herbizide wie Metflurazon, Norflurazon, Flufenican, Diflufenican, Picolinafen, Beflubutamid, Fluridone, Flurochloridone, Flurtamone, Mesotrione, Sulcotrione, Isoxachlortole, Isoxaflutole, Benzofenap, Pyrazolynate, Pyrazoxyfen, Benzobicyclon, amitrole, clomazone, Aclonifen, 4-(3-trifluormethylphenoxy)- 2-(4-trifluoromethylphenyl)pyrimidin, und 3-heterocyclyl-substituierte Benzoylderivate der Formel (vgl. WO-A-96/26202, WO-A-97/41116, WO-A-97/41117 und WO-A-97/41118) 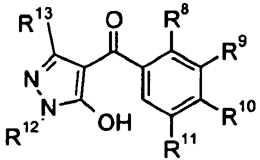 worin die Substituenten R⁸ bis R¹³ folgende Bedeutung haben:
R⁸, R¹⁰ Wasserstoff, Halogen, C₁-C₆-Alkyl, C₁-C₆-Haloalkyl, C₁-C₆-Alkoxy, C₁-C₆-Haloalkoxy, C₁-C₆-Alkylthio, C₁-C₆-Alkylsulfinyl oder C₁-C₆-Alkylsulfonyl;
R⁹ bedeutet ein heterocyclisches Radikal aus der Gruppe bestehend aus Thiazol-2-yl, thiazol-4-yl, Thiazol-5-yl, Isoxazol-3-yl, Isoxazol-4-yl, Isoxazol-5-yl, 4,5-dihydroisoxazol-3-yl, 4,5-dihydroisoxazol-4-yl und 4,5-dihydroisoxazol-5-yl, worin die genannten Radikale einen oder mehrere Substituenten tragen können z.B. mono-, di-, tri- or tetrasubstituiert sein können durch Halogen, C₁-C₄-Alkyl, C₁-C₄-Alkoxy, C₁-C₄-Haloalkyl, C₁-C₄-Haloalkoxy oder C₁-C₄-Alkylthio;
R" = Wasserstoff, Halogen oder C₁-C₆-Alkyl;
R¹² = C₁-C₆-Alkyl;
R¹³ = Wasserstoff oder C₁-C₆-Alkyl.

Weitere geeignete Herbizide sind EPSP-Synthase-Inhibitoren wie Glyphosat;
Glutamin-Synthase-Inhibitoren wie Glufosinate und Bilanaphos;
DHP-Synthase-Inhibitoren wie Asulam;
Mitose-Inhibitoren wie Benfluralin, Butralin, Dinitramine, Ethalfluralin, Fluchloralin, Isopropalin, Methalpropalin, Nitralin, Oryzalin, Pendimethalin, Prodiamine, Profluralin, Trifluralin, Amiprofos-methyl, Butamifos, Dithiopyr, Thiazopyr, Propyzamide, Tebutam, Chlorthal, Carbetamide, Chlorbufam, Chlorpropham and Propham;
VLCFA-Inhibitoren wie Acetochlor, Alachlor, Butachlor, Butenachlor, Delachlor, Diethatyl, Dimethachlor, Dimethenamid, Dimethenamid-P, Metazachlor, Metolachlor, S-Metolachlor, Pretilachlor, Propachlor, Propisochlor, Prynachlor, Terbuchlor, Thenylchlor, Xylachlor, Allidochlor, CDEA, Epronaz, Diphenamid, Napropamide, Naproanilide, Pethoxamid, Flufenacet, Mefenacet, Fentrazamide, Anilofos, Piperophos, Cafenstrole, Indanofan und Tridiphan;
Inhibitoren für die Biosynthese von Cellulose wie Dichlobenil, Chlorthiamid, Isoxaben und Flupoxam;
Herbizide wie Dinofenat, Dinoprop, Dinosam, Dinoseb, Dinoterb, DNOC, Etinofen und Medinoterb;
Auxin-Herbizide wie Clomeprop, 2,4-D, 2,4,5-T, MCPA, MCPA Thioethyl, Dichlorprop, Dichlorprop-P, Mecoprop, Mecoprop-P, 2,4-DB, MCPB, Chloramben, Dicamba, 2,3,6-TBA, Tricamba, Quinclorac, Quinmerac, Clopyralid, Fluroxypyr, Picloram, Triclopyr und Benazolin;
Auxin-Transport-Inhibitoren wie Naptalam, Diflufenzopyr;
außerdem: Benzoylprop, Flamprop, Flamprop-M, Bromobutide, Chlorflurenol, Cinmethylin, Methyldymron, Etobenzanid, Fosamine, Metam, Pyributicarb, Oxaziclomefone, Dazomet, Triaziflam und Methyl bromide.

Der Begriff "Safener" hat die folgende Bedeutung: Es ist bekannt, dass in einigen Fällen bessere Herbizidverträglichkeit durch die gemeinsame Applikation spezifisch wirkender Herbizide mit organischen aktiven Verbindungen erreicht werden kann, welche selber herbizid wirken können. In diesen Fällen wirken diese Verbindungen als Antidot oder Antagonist und werden aufgrund der Tatsache, dass sie Schaden von Nutzpflanzen reduzieren bzw. verhindern als "Safener" bezeichnet.

Die folgende Liste zeigt mögliche Safener auf, soll aber nicht auf diese beschränkt sein:
Benoxacor, Cloquintocet, Cyometrinil, Dichlormid, Dicyclonon, Dietholate, Fenchlorazole, Fenclorim, Flurazole, Fluxofenim, Furilazole, Isoxadifen, Mefenpyr, Mephenate, Naphthalic Anhydride, 2,2,5-Trimethyl-3-(dichloroacetyl)-1,3-oxazolidine (R-29148), 4-(dichloroacetyl)-1-oxa-4-azaspiro[4.5]decane (AD-67; MON 4660) und Oxabetrinil.

Die folgende Liste von Verbindungen mit wachstumsregulatorischer Wirkung zeigt mögliche Wirkstoffe auf, soll aber nicht auf diese beschränkt sein:
1-Naphthylacetamid, 1-Naphthylessigsäure, 2-Naphthyloxyessigsäure, 3-CPA, 4-CPA, Ancymidol, Anthrachinon, BAP, Butifos; Tribufos, Butralin, Chlorflurenol, Chlormequat, Clofencet, Cyclanilide, Daminozide, Dicamba, Dikegulac sodium, Dimethipin, Chlorfenethol, Etacelasil, Ethephon, Ethychlozate, Fenoprop, 2,4,5-TP, Fluoridamid, Flurprimidol, Flutriafol, Gibberellic acid, Gibberillin, Guazatin, Imazalil, Indolylbuttersäure, Indolylessigsäure, Karetazan, Kinetin, Lactidichlor-ethyl, Maleic hydrazide, Mefluidide, Mepiquat-chlorid, Naptalam, Paclobutrazole, Prohexadione calcium, Quinmerac, Sintofen, Tetcyclacis, Thidiazuron, Triiodobezoicacid, Triapenthenol, Triazethan, Tribufos, Trinexapacethyl,Uniconazole.
- 2-Methoxybenzophenone, wie sie in EP-A 897904 durch die allgemeine Formel I beschrieben werden, z.B. Metrafenon;
- 6-Aryl-[1,2,4]triazolo[1,5-a]pyrimidine wie sie z.B. in WO 98/46608, WO 99,41255 oder WO 03/004465 jeweils durch die allgemeine Formel I beschrieben werden;

Beispiele für Herbizide, die als erfindungsgemäße wässrige Wirkstoffzusammensetzung formuliert werden können, umfassen:
- 1,3,4-Thiadiazole wie Buthidazole und Cyprazole;
- Amide wie Allidochlor, Benzoylpropethyl, Bromobutide, Chlorthiamid, Dimepiperate, Dimethenamid, Diphenamid, Etobenzanid, Flampropmethyl, Fosamin, Isoxaben, Metazachlor, Alachlor, Acetochlor, Metolachlor, Monalide, Naptalame, Pronamid, Propanil;
- Aminophosphorsäuren wie Bilanafos, Buminafos, Glufosinateammonium, Glyphosate, Sulfosate;
- Aminotriazole wie Amitrol, Anilide wie Anilofos, Mefenacet;
- Aryloxyalkan-säure wie 2,4-D, 2,4-DB, Clomeprop, Dichlorprop, Dichlorprop-P, Dichlorprop-P, Fenoprop, Fluroxypyr, MCPA, MCPB, Mecoprop, Mecoprop-P, Napropamide, Napro-panilide, Triclopyr;
- Benzoesäuren wie Chloramben, Dicamba;
- Benzothiadiazinone wie Bentazon;
- Bleacher wie Clomazone, Diflufenican, Fluorochloridone, Flupoxam, Fluridone, Pyrazolate, Sulcotrione;
- Carbamate wie Carbetamid, Chlorbufam, Chlorpro-pham, Desmedipham, Phenmedipham, Vernolate;
- Chinolinsäuren wie Quinclorac, Quinmerac;
- Dichlorpropionsäuren wie Dalapon;
- Dihydrobenzofurane wie Ethofumesate;
- Dihydrofuran-3-on wie Flurtamone;
- Dinitroaniline wie Benefin, Butralin, Dinitramin, Ethalfluralin, Fluchloralin, Isopropalin, Nitralin, Oryzalin, Pendimethalin, Prodiamine, Profluralin, Trifluralin, Dinitrophenole wie Bromofenoxim, Dinoseb, Dinoseb-acetat, Dinoterb, DNOC, Minoterb-Acetat;
- Diphenylether wie Acifluorfen-sodium, Aclonifen, Bifenox, Chlornitrofen, Difenoxuron, Ethoxyfen, Fluorodifen, Fluoroglycofen-ethyl, Fomesafen, Furyloxyfen, Lactofen, Nitrofen, Nitrofluorfen, Oxyfluorfen;
- Dipyridyle wie Cyperquat, Difenzoquat-methylsulfat, Diquat, Paraquat-dichlorid;
- Imidazole wie Isocarbamid;
- Imidazolinone wie Imazamethapyr, Imazapyr, Imazaquin, Imazethabenzmethyl, Imazethapyr, Imazapic, Imazamox;
- Oxadiazole wie Methazole, Oxadiargyl, Oxadiazon;
- Oxirane wie Tridiphane;
- Phenole wie Bromoxynil, loxynil;
- Phenoxyphenoxypropionsäureester wie Clodinafop, Cyhalofop-butyl, Diclofopmethyl, Fenoxaprop-ethyl, Fenoxaprop-p-ethyl, Fenthiapropethyl, Fluazifopbutyl, Fluazifop-p-butyl, Haloxyfop-ethoxy-ethyl, Haloxyfop-methyl, Haloxyfop-p-methyl, Isoxapyrifop, Propaquizafop, Quizalofop-ethyl, Quizalofop-p-ethyl, Quizalofop-tefuryl;
- Phenylessigsäuren wie Chlorfenac;
- Phenylpropionsäuren wie Chlorophenprop-methyl;
- ppi-Wirkstoffe wie Benzofenap, Cinidon-Ethyl, Flumiclorac-pentyl, Flumioxazin, Flumipropyn, Flupropacil, Pyrazoxyfen, Sulfentrazone, Thidiazimin;
- Pyrazole wie Nipyraclofen;
- Pyridazine wie Chloridazon, Maleic hydrazide, Norflurazon, Pyridate;
- Pyridincarbonsäuren wie Clopyralid, Dithiopyr, Picloram, Thiazopyr;
- Pyrimidylether wie Pyrithiobacsäure, Pyrithiobac-sodium, KIH-2023, KIH-6127;
- Sulfonamide wie Flumetsulam, Metosulam;
- Triazolcarboxamide wie Triazofenamid;
- Uracile wie Bromacil, Lenacil, Terbacil;
- ferner Benazolin, Benfuresate, Bensulide, Benzofluor, Bentazon, Butamifos, Cafenstrole, Chlorthal-dimethyl, Cinmethylin, Dichlobenil, Endothall, Fluorbentranil, Mefluidide, Perfluidone, Piperophos, Topramezone und Prohexandion-Calcium;
- Sulfonylharnstoffe wie Amidosulfuron, Azimsulfuron, Bensulfuron-methyl, Chlorimuron-ethyl, Chlorsulfuron, Cinosulfuron, Cyclosulfamuron, Ethametsulfuron-methyl, Flazasulfuron, Halosulfuron-methyl, Imazosulfuron, Metsulfuron-methyl, Nicosulfuron, Primisulfuron, Prosulfuron, Pyrazosulfuronethyl, Rimsulfuron, Sulfometuron-methyl, Thifensulfuron-methyl, Triasulfuron, Tribenuron-methyl, Triflusulfuron-methyl, Tritosulfuron;
- Pflanzenschutz-Wirkstoffe vom Cyclohexenon-Typ wie Alloxydim, Clethodim, Cloproxydim, Cycloxydim, Sethoxydim und Tralkoxydim. Ganz besonders bevorzugte herbizide Wirkstoffe vom Cyclohexenon-Typ sind: Tepraloxydim (vgl. AGROW, Nr. 243, 3.11.95, Seite 21, Caloxydim) und 2-(1-[2-{4-Chlorphenoxy}propyl-oxyimino]butyl)-3-hydroxy-5-(2H-tetrahydrothiopyran-3-yl)-2-cyclohexen-1-on und vom Sulfonylharnstoff-Typ: N-(((4-methoxy-6-[trifluormethyl]-1,3,5-triazin-2-yl)amino)carbo-nyl)-2-(trifluormethyl)-benzolsulfonamid.

Beispiele für Insektizide, die als erfindungsgemäße wässrige Wirkstoffzusammensetzung formuliert werden können, umfassen:
- Pyrethroide wie Bifenthrin, Cyfluthrin, Cycloprothrin, Cypermethrin, Deltamethrin, Esfenvalerate, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, alpha-Cypermethrin, Zeta-Cypermethrin, Permethrin;
- N-Phenylsemicarbazone, wie sie in EP-A 462 456 durch die allgemeine Formel I beschrieben werden, insbesondere Verbindungen der allgemeinen Formel IV
- 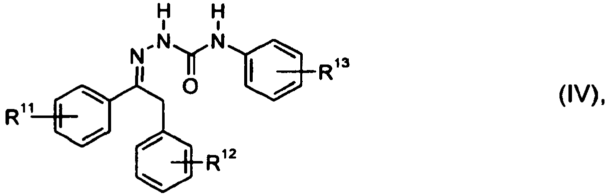 ○ worin R11 und R12 unabhängig voneinander für Wasserstoff, Halogen, CN, C1-C4-Alkyl, C1-C4-Alkoxy, C1-C4-Haloalkyl oder C1-C4-Haloalkoxy stehen und R13 für C1-C4-Alkoxy, C1-C4-Haloalkyl oder C1-C4-Haloalkoxy steht, z.B. Verbindung IV, worin R1 für 3-CF3 und R2 für 4-CN stehen und R3 4-OCF3 bedeutet.

Brauchbare Wachstumsregulatoren sind z.B. Chlormequat-chlorid, Mepiquatchlorid, Prohexadion-Calcium oder die die Gruppe der Gibberelline. Dazu gehören z.B. die Gibberelline GA1, GA3, GA4, GA5 und GA7 etc. und die entsprechenden exo-16,17-Dihydrogibberelline sowie die Derivate davon, z.B. die Ester mit C1-C4-Carbonsäuren. Erfindungsgemäß bevorzugt ist das exo-16,17-Dihydro-GA5-13-acetat.

Eine bevorzugte Ausführungsform der Erfindung betrifft die Verwendung der erfindungsgemäßen amphiphilen Polymerzusammensetzungen zur Herstellung wässriger Wirkstoffzusammensetzungen von Fungiziden, insbesondere Strobilurinen, Azolen und 6-Aryltriazolo[1,5a]pyrimidinen, wie sie z.B. in WO 98/46608, WO 99/41255 oder WO 03/004465 jeweils durch die allgemeine Formel I beschrieben werden, insbesondere für Wirkstoffe der allgemeinen Formel V, 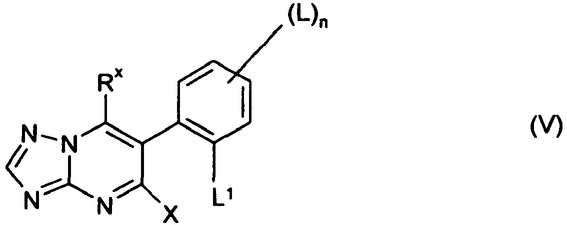 worin:
- Rx: für eine Gruppe NR14R15 steht, oder lineares oder verzweigtes C1-C8-Alkyl, das gegebenenfalls durch Halogen, OH, C1-C4-Alkoxy, Phenyl oder C3-C6-Cycloalkyl substiuiert ist, C2-C6-Alkenyl, C3-C6-Cycloalkyl, C3-C6-Cycloalkenyl, Phenyl oder Naphthyl, wobei die 4 zuletzt genannten Reste 1, 2, 3 oder 4 Substituenten ausgewählt unter Halogen, OH, C1-C4-Alkyl, C1-C4-Halogenalkoxy, C1-C4-Alkoxy und C1-C4-Halogenalkyl aufweisen können;
- R14, R15: unabhängig voneinander für Wasserstoff, C1-C8-Alkyl, C1-C8-Halogenalkyl, C3-C10-Cycloalkyl, C3-C6-Halogencycloalkyl, C2-C8-Alkenyl, C4-C10-Alkadienyl, C2-C8-Halogenalkenyl, C3-C6-Cycloalkenyl, C2-C8-Halogencycloalkenyl, C2-C8-Alkinyl, C2-C8-Halogenalkinyl oder C3-C6-Cycloalkinyl,
- R14 und R15: zusammen mit dem Stickstoffatom, an das sie gebunden sind, fünf- bis achtgliedriges Heterocyclyl, welches über N gebunden ist und ein, zwei oder drei weitere Heteroatome aus der Gruppe O, N und S als Ringglied enthalten und/oder einen oder mehrere Substituenten aus der Gruppe Halogen, C1-C6-Alkyl, C1-C6-Halogenalkyl, C2-C6-Alkenyl, C2-C6-Halogenalkenyl, C1-C6-Alkoxy, C1-C6-Halogenalkoxy, C3-C6-Alkenyloxy, C3-C6-Halogenalkenyloxy, (exo)-C1-C6-Alkylen und Oxy-C1-C3-alkylenoxy tragen kann;
- L: ausgewählt ist unter Halogen, Cyano, C1-C6-Alkyl, C1-C4-Halgoenalkyl, C1-C6-Alkoxy, C1-C4-Halgoenalkoxy und C1-C6-Alkoxycarbonyl;
- L1: Halogen, C1-C6-Alkyl oder C1-C6-Halogenalkyl und insbesondere Fluor oder Chlor bedeutet;
- X: für Halogen, C1-C4-Alkyl, Cyano, C1-C4-Alkoxy oder C1-C4-Halogenalkyl und vorzugsweise für Halogen oder Methyl steht und insbesondere Chlor bedeutet.

Beispiele für Verbindungen der Formel V sind
5-Chlor-7-(4-methylpiperidin-1-yl)-6-(2,4,6-trifluorphenyl)-[1,2,4]triazolo[1,5-a]pyrimidin,
5-Chlor-7-(4-methylpiperazin-1-yl)-6-(2,4,6-trifluorphenyl)-[1,2,4]triazolo[1,5-a]pyrimidin, 5-Chlor-7-(morpholin-1-yl)-6-(2,4,6-trifluorphenyl)-[1,2,4]triazolo[1,5-a]pyrimidin,
5-Chlor-7-(piperidin-1-yl)-6-(2,4,6-trifluorphenyl)-[1,2,4]triazolo[1,5-a]pyrimidin,
5-Chlor-7-(morpholin-1-yl)-6-(2,4,6-trifluorphenyl)-[1,2,4]triazolo[1,5-a]pyrimidin,
5-Chlor-7-(isopropylamino)-6-(2,4,6-trifluorphenyl)-[1,2,4]triazolo[1,5-a]pyrimidin,
5-Chlor-7-(cyclopentylamino)-6-(2,4,6-trifluorphenyl)-[1,2,4]triazolo[1,5-a]pyrimidin,
5-Chlor-7-(2,2,2-trifluorethylamino)-6-(2,4,6-trifluorphenyl)-[1,2,4]triazolo[1,5-a]pyrimidin,
5-Chlor-7-(1,1,1-trifluorpropan-2-ylamino)-6-(2,4,6-trifluorphenyl)-[1,2,4]triazolo[1,5-a]pyrimidin,
5-Chlor-7-(3, 3-dimethylbutan-2-ylamino)-6-(2,4,6-trifluorphenyl)-[1,2,4]triazolo[1,5-a]pyrimidin,
5-Chlor-7-(cyclohexylmethyl)-6-(2,4,6-trifluorphenyl)-[1,2,4]triazolo[1,5-a]pyrimidin,
5-Chlor-7-(cyclohexyl)-6-(2,4,6-trifluorphenyl)-[1,2,4]triazolo[1,5-a]pyrimidin,
5-Chlor-7-(2-methylbutan-3-yl)-6-(2,4,6-trifluorphenyl)-[1,2,4]triazolo[1,5-a]pyrimidin,
5-Chlor-7-(3-methylpropan-1-yl)-6-(2,4,6-trifluorphenyl)-[1,2,4]triazolo[1,5-a]pyrimidin,
5-Chlor-7-(4-methylcyclohexan-1-yl)-6-(2,4,6-trifluorphenyl)-[1,2,4]triazolo[1,5-a]pyrimidin,
5-Chlor-7-(hexan-3-yl)-6-(2,4,6-trifluorphenyl)-[1,2,4]triazolo[1,5-a]pyrimidin,
5-Chlor-7-(2-methylbutan-1-yl)-6-(2,4,6-trifluorphenyl)-[1,2,4]triazolo[1, 5-a]pyrimidin,
5-Chlor-7-(3-methylbutan-1-yl)-6-(2,4,6-trifluorphenyl)-[1,2,4]triazolo[1,5-a]pyrimidin,
5-Chlor-7-(1-methylpropan-1-yl)-6-(2,4,6-trifluorphenyl)-[1,2,4]triazolo[1,5-a]pyrimidin,
5-Methyl-7-(4-methylpiperidin-1-yl)-6-(2,4,6-trifluorphenyl)-[1,2,4]triazolo[1,5-a]pyrimidin,
5-Methyl-7-(4-methylpiperazin-1-yl)-6-(2,4,6-trifluorphenyl)-[1,2,4]triazolo[1,5-a]pyrimidin,
5-Methyl-7-(morpholin-1-yl)-6-(2,4,6-trifluorphenyl)-[1,2,4]triazolo[1,5-a]pyrimidin,
5-Methyl-7-(piperidin-1-yl)-6-(2,4,6-trifluorphenyl)-[1,2,4]triazolo[1,5-a]pyrimidin,
5-Methyl-7-(morpholin-1-yl)-6-(2,4,6-trifluorphenyl)-[1,2,4]triazolo[1,5-a]pyrimidin,
5-Methyl-7-(isopropylamino)-6-(2,4,6-trifluorphenyl)-[1,2,4]triazolo[1,5-a]pyrimidin,
5-Methyl-7-(cyclopentylamino)-6-(2,4,6-trifluorphenyl)-[1,2,4]triazolo[1,5-a]pyrimidin,
5-Methyl-7-(2,2,2-trifluorethylamino)-6-(2,4,6-trifluorphenyl)-[1,2,4]triazolo[1,5-a]pyrimidin,
5-Methyl-7-(1,1,1-trifluorpropan-2-ylamino)-6-(2,4,6-trifluorphenyl)-[1,2,4]triazolo[1,5-a]pyrimidin,
5-Methyl-7-(3,3-dimethylbutan-2-ylamino)-6-(2,4,6-trifluorphenyl)-[1,2,4]triazolo[1,5-a]pyrimidin,
5-Methyl-7-(cyclohexylmethyl)-6-(2,4,6-trifluorphenyl)-[1,2,4]triazolo[1,5-a]pyrimidin,
5-Methyl-7-(cyclohexyl)-6-(2,4,6-trifluorphenyl)-[1,2,4]triazolo[1,5-a]pyrimidin,
5-Methyl-7-(2-methylbutan-3-yl)-6-(2,4,6-trifluorphenyl)-[1,2,4]triazolo[1,5-a]pyrimidin,
5-Methyl-7-(3-methylpropan-1-yl)-6-(2,4,6-trifluorphenyl)-[1,2,4]triazolo[1,5-a]pyrimidin,
5-Methyl-7-(4-methylcyclohexan-1-yl)-6-(2,4, 6-trifluorphenyl)-[1,2,4]triazolo[1,5-a]pyrimidin,
5-Methyl-7-(hexan-3-yl)-6-(2,4,6-trifluorphenyl)-[1,2,4]triazolo[1,5-a]pyrimidin,
5-Methyl-7-(2-methylbutan-1-yl)-6-(2,4,6-trifluorphenyl)-[1,2,4]triazolo[1,5-a]pyrimidin,
5-Methyl-7-(3-methylbutan-1-yl)-6-(2,4,6-trifluorphenyl)-[1,2,4]triazolo[1,5-a]pyrimidin und 5-Methyl-7-(1-methylpropan-1-yl)-6-(2,4,6-trifluorphenyl)-[1,2,4]triazolo[1,5-a]pyrimidin.

Eine weitere bevorzugte Ausführungsform der Erfindung betrifft die Verwendung der erfindungsgemäßen amphiphilen Polymerzusammensetzungen zur Herstellung wässriger Wirkstoffzusammensetzungen von Insektiziden, insbesondere von Arylpyrrolen wie Chlorfenapyr, von Pyrethroiden wie Bifenthrin, Cyfluthrin, Cycloprothrin, Cypermethrin, Deltamethrin, Esfenvalerate, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, alpha-Cypermethrin, Zeta-Cypermethrin und Permethrin, von Neonicotinoiden und von Semicarbazonen der Formel IV, von Fipronil.

Die oben genannten Pestizide werden gegen die entsprechenden Schädlinge eingesetzt.

### Schädlinge

Bekämpfung unerwünschten Pflanzenwuchses bedeutet die Bekämpfung/Zerstörung von Planzen, welche an Orten wachsen, an welchen sie unerwünscht sind, z.B. von

Dicotyledonen Pflanzen der Arten: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Monocotyledonen Pflanzen der Arten: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, Apera

Der Begriff unerwünschte Insekten- oder Milben beschreibt ist aber nicht beschränkt auf folgende Gattungen:

Lepidoptera, zum Beispiel Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,

Käfer (Coleoptera) zum Beispiel Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, lps typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,

Diptera, zum Beispiel Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa,

Thysanoptera zum Beispiel Frankliniella fusca, Frankliniella occidentalis, Frankliniella tritici, Scirtothrips citri, Thrips oryzae, Thrips palmi und Thrips tabaci,

Hymenoptera zum Beispiel Athalia rosae, Atta cephalotes, Atta sexdens, Atta texana, Hoplocampa minuta, Hoplocampa testudinea, Monomorium pharaonis, Solenopsis geminata und Solenopsis invicta,

Heteroptera zum Beispiel Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,

Homoptera zum Beispiel Acyrthosiphon onobrychis, Adelges laricis, Aphidula nasturtii, Aphis fabae, Aphis forbesi, Aphis pomi, Aphis gossypii, Aphis grossulariae, Aphis schneideri, Aphis spiraecola, Aphis sambuci, Acyrthosiphon pisum, Aulacorthum solani, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzodes persicae, Myzus ascalonicus, Myzus cerasi, Myzus varians, Nasonovia ribis-nigri, Nilaparvata lugens, Pemphigus bursarius, Perkinsiella saccharicida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Trialeurodes vaporariorum, Toxoptera aurantiiund, und Viteus vitifolii;

Termiten (Isoptera), z.B. Calotermes flavicollis, Leucotermes flavipes, Reticulitermes lucifugus und Termes natalensis;

Orthoptera, z.B. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus ;

Arachnoidea, zum Beispiel Acarina, z.B. aus den Familien Argasidae, Ixodidae und Sarcoptidae, z.B. Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Omithodorus moubata, Otobius megnini, Dermanyssus gallinae, Psoroptes ovis, Rhipicephalus appendiculatus, Rhipicephalus evertsi, Sarcoptes scabiei, und Eriophyidae spp. z.B. Aculus schlechtendali, Phyllocoptrata oleivora und Eriophyes sheldoni; Tarsonemidae spp. z.B. Phytonemus pallidus und Polyphagotarsonemus latus; Tenuipalpidae spp. z.B. Brevipalpus phoenicis; Tetranychidae spp. z.B. Tetranychus cinnabarinus, Tetranychus kanzawai, Tetranychus pacificus, Tetranychus telarius und Tetranychus urticae, Panonychus ulmi, Panonychus citri, und oligonychus pratensis;

Nematoden, insbesonders Pflanzen parasitäre Nematoen, z.B.plant root knot nematodes, Meloidogyne hapla, Meloidogyne incognita, Meloidogyne javanica, und andere Meloidogyne species; cyst-forming nematodes, Globodera rostochiensis und andere Globodera species; Heterodera avenae, Heterodera glycines, Heterodera schachtii, Heterodera trifolii, und andere Heterodera species; Seed gall nematodes, Anguina species; Stem und foliar nematodes, Aphelenchoides species; Sting nematodes, Belonolaimus longicaudatus und andere Belonolaimus species; Pine nematodes, Bursaphelenchus xylophilus und andere Bursaphelenchus species; Ring nematodes, Criconema species, Criconemella species, Criconemoides species, Mesocriconema species; Stem und bulb nematodes, Ditylenchus destructor, Ditylenchus dipsaci und andere Ditylenchus species; Awl nematodes, Dolichodorus species; Spiral nematodes, Heliocotylenchus multicinctus und andere Helicotylenchus species; Sheath und sheathoid nematodes, Hemicycliophora species und Hemicriconemoides species; Hirshmanniella species; Lance nematodes, Hoploaimus species; false rootknot nematodes, Nacobbus species; Needle nematodes, Longidorus elongatus und andere Longidorus species; Lesion nematodes, Pratylenchus neglectus, Pratylenchus penetrans, Pratylenchus curvitatus, Pratylenchus goodeyi und andere Pratylenchus species; Burrowing nematodes, Radopholus similis und andere Radopholus species; Reniform nematodes, Rotylenchus robustus und andere Rotylenchus species; Scutellonema species; Stubby root nematodes, Trichodorus primitivus und andere Trichodorus species, Paratrichodorus species; Stunt nematodes, Tylenchorhynchus claytoni, Tylenchorhynchus dubius und andere Tylenchorhynchus species; Citrus nematodes, Tylenchulus species; Dagger nematodes, Xiphinema species ;
sowie Reis pathogene wie
und rice pathogens z.B. rice water weevil (Lissorhoptrus oryzaphilus), rice stem borer (Chilo suppresalis), rice leaf roller, rice leaf beetle, rice leaf miner (Agromyca oryzae), leafhoppers (Nephotettix spp.;especially smaller brown leafhopper, green rice leafhopper), planthoppers (Delphacidae; especially white backed planthopper, brown rice planthopper), stinkbugs;

Der Begriff phytopathogene Pilze beschreibt ist aber nicht beschränkt auf folgende Spezies:

Blumeria graminis (echter Mehltau) an Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis an Äpfeln, Bipolaris- und Drechslera-Arten an Getreide, Reis und Rasen, Septoria nodorum an Weizen, Botrytis cinerea an Erdbeeren, Gemüse, Zierpflanzen und Reben, Mycosphaerella-Arten an Bananen, Erdnüssen und Getreide, Pseudocercosporella herpotrichoides an Weizen und Gerste, Pyricularia oryzae an Reis, Phytophthora infestans an Kartoffeln und Tomaten, Pseudoperonospora-Arten an Kürbisgewächsen und Hopfen, Plasmopara viticola an Reben, Alternaria-Arten an Gemüse und Obst sowie Fusarium- und Verticillium-Arten, Bipolaris- und Drechslera-Arten sowie Pyricularia oryzae, Corticium sasakii (syn. Rhizoctonia solani) und Cochliobolus miyabeanus an Reispflanzen und ggf. an deren Saatgut, Paecilomyces variotii an Materialien wie Holz.

Außerdem eignen sich die erfindungsgemäßen amphiphilen Polymerzusammensetzungen zur Herstellung wässriger Wirkstoffzusammensetzungen von pharmazeutischen Wirkstoffen und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Außerdem eignen sich die erfindungsgemäßen amphiphilen Polymerzusammensetzungen zur Herstellung wässriger Zubereitungen von kosmetischen Wirkstoffen, insbesondere von kosmetischen Öle und Fetten wie Erdnußöl, Jojobaöl, Kokosnußöl, Mandelöl, Olivenöl, Palmöl, Ricinusöl, Sojaöl oder Weizenkeimöl, etherische Öle wie Latschenkiefernöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Salbeiöl, Wacholderöl, Zitronenöl, Anisöl, Kardamonöl; Pfefferminzöl, Campheröl etc. oder für Mischungen aus diesen Ölen.

Außerdem eignen sich die erfindungsgemäßen amphiphilen Polymerzusammensetzungen zur Herstellung wässriger Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat und Vitamin K.

Außerdem eignen sich die erfindungsgemäßen amphiphilen Polymerzusammensetzungen zur Herstellung, bevorzugt wäßriger, Zubereitungen zu Saatgutbehandlung.

Beispiele für Formulierungstypen sind emulgierbare Konzentrate, Suspensionen, lösliche Konzentrate, dispergierbare Konzentrate, Pasten, Pastillen, benetzbare Pulver, Stäube (DP) oder Granulate (GR, FG, GG, MG), die entweder in Wasser löslich oder dispergierbar sein können, zu nennen. Gängige Formulierungstypen für die Saatgutbehandlung sind FS (flowable concentrates), LS (solutions), DS (powders for dry treatment), WS (water dispersible powders for slurry treatment), SS (watersoluble powders SS) und ES (emulsion). Die Herstellung dieser Formulierungen sowie die dafür benötigte Technologie ist dem Fachmann bekannt (vgl. US 3,060,084, EP-A 707 445 (für flüssige Konzentrate), Browning, "Agglomeration", Chemical Engineering, Dec. 4, 1967, 147-48, Perry's Chemical Engineer's Handbook, 4th Ed., McGraw-Hill, New York, 1963, S. 8-57 und ff. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 und Mollet, H., Grubemann, A., Formulation technology, Wiley VCH Verlag GmbH, Weinheim (Federal Republic of Germany), 2001), 2. D. A. Knowles, Chemistry and Technology of Agrochemical Formulations, Kluwer Academic Publishers, Dordrecht, 1998 (ISBN 0-7514-0443-8).

Die vorliegende Erfindung umfasst auch Saatgut, welches mit Pestizide enthaltenden wässrigen Polymerdispersionen behandelt wurde. Bei der Saatgutbehandlung werden im allgemeinen Aufwandmengen an Pestiziden von 0,1 bis 10kg/100 kg Saatgut, vorzugsweise 1 bis 5kg/100 kg, insbesondere 1 bis 2,5kg/100 kg verwendet.

Das oder die Monomere (D), das bzw. die optional in die im erfindungsgemäßen Verfahren eingesetzten Polymerisate einpolymerisiert werden können, sind von (A) verschieden. Als bevorzugte Monomere (D) sind zu nennen:

Ethylenisch ungesättigte C3-C8-Carbonsäurederivate der allgemeinen Formel II

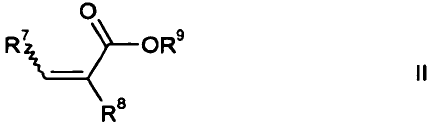

Acrylamide der Formel III, 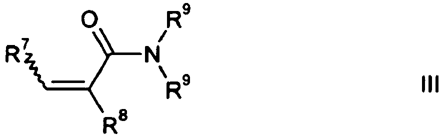 nicht-cyclische Amide der allgemeinen Formel IV a und cyclische Amide der allgemeinen Formel IV b 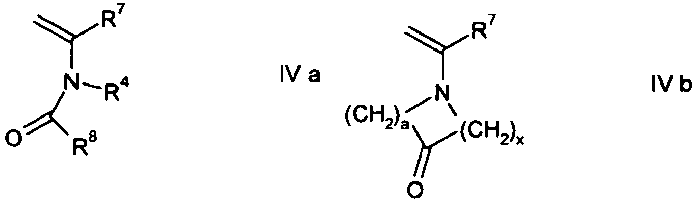 C1-C20-Alkylvinylether wie Methylvinylether, Ethylvinylether, n-Propylvinylether, Isopropylvinylether, n-Butylvinylether, Isobutylvinylether, 2-Ethylhexylvinylether oder n-Octadecylvinylether;
N-Vinyl-Derivate von stickstoffhaltigen aromatischen Verbindungen, bevorzugt N-Vinylimidazol, 2-Methyl-1-vinylimidazol, N-Vinyloxazolidon, N-Vinyltriazol, 2-Vinylpyridin, 4-Vinylpyridin, 4-Vinylpyridin-N-oxid, N-Vinylimidazolin, N-Vinyl-2-methylimidazolin,
alkoxylierte ungesättigte Ether der allgemeinen Formel V,

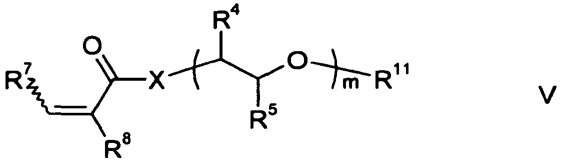

Ester und Amide der allgemeinen Formel VI, 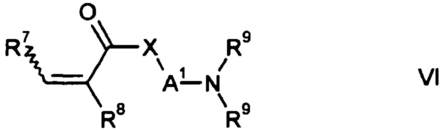 ungesättigte Ester der allgemeinen Formel VII

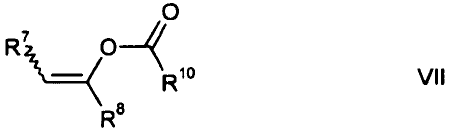

Weiterhin geeignet sind Sulfonat-, Phosphat- oder Phosphonat-Gruppen-haltige Monomere, wie beispielsweise Vinylsulfonsäure und Vinylphosphonsäure und Verbindungen der allgemeinen Formel IX 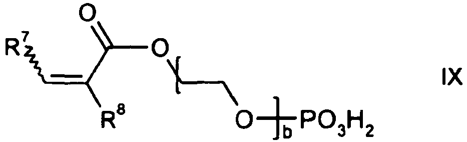 wobei Phosphat-Gruppen, Sulfonat-Gruppen oder Phosphonatgruppen gegebenenfalls partiell oder vollständig in Form von Alkalimetallsalzen vorliegen können.
wobei die Variablen wie folgt definiert sind:
- R7,: gewählt aus unverzweigten oder verzweigten C1-C10-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethyl-propyl, iso-Amyl, n-Hexyl, iso-Hexyl, sec.-Hexyl, n-Heptyl, n-Octyl, n-Nonyl, n-Decyl; besonders bevorzugt C1-C4-Alkyl wie Methyl, Ethyl, n-Propyl, iso-Propyl, n-Butyl, iso-Butyl, sec.-Butyl und tert.-Butyl, und insbesondere Wasserstoff;
- R8: gewählt aus unverzweigten oder verzweigten C1-C10-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethyl-propyl, iso-Amyl, n-Hexyl, iso-Hexyl, sec.-Hexyl, n-Heptyl, n-Octyl, n-Nonyl, n-Decyl; besonders bevorzugt C1-C4-Alkyl wie Methyl, Ethyl, n-Propyl, iso-Propyl, n-Butyl, iso-Butyl, sec.-Butyl und tert.-Butyl, insbesondere Methyl und insbesondere Wasserstoff;
- R9: gleich oder verschieden und C1-C22-Alkyl, verzweigt oder unverzweigt, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl, n-Dodecyl, n-Eicosyl; besonders bevorzugt C1-C4-Alkyl wie Methyl, Ethyl, n-Propyl, iso-Propyl, n-Butyl, iso-Butyl, sec.-Butyl und tert.-Butyl oder Wasserstoff; mit der Maßgabe, daß in der Formel II R9 nicht für Wasserstoff steht;
- A1: gleich oder verschieden und C2-C6-Alkylen, beispielsweise -CH2-, -CH(CH3)-, - (CH2)2-, -CH2-CH(CH3)-, -(CH2)3-, -CH2-CH(C2H5)-, -(CH2)4-, -(CH2)5-, -(CH2)6-, vorzugsweise C1-C3-Alkylen; insbesondere - (CH2)2-, -CH2-CH(CH3)- und -CH2-CH(C2H5)-;
- x: eine ganze Zahl im Bereich von 2 bis 6, vorzugsweise 3 bis 5;
- a: eine ganze Zahl im Bereich von 0 bis 6, vorzugsweise im Bereich von 0 bis 2;
- b: eine ganze Zahl im Bereich von 1 bis 40, bevorzugt 1 bis 10,
- m: eine ganze Zahl im Bereich von 2 bis 200, bevorzugt 10 bis 40;
- R10, R11: gleich oder verschieden und gewählt aus Wasserstoff, unverzweigten oder verzweigten C1-C10-Alkyl und wobei unverzweigtes und verzweigtes C1-C10-Alkyl wie oben stehend definiert ist;
- X: Sauerstoff oder N-R12;
- R12: gewählt aus unverzweigten oder verzweigten C1-C10-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethyl-propyl, iso-Amyl, n-Hexyl, iso-Hexyl, sec.-Hexyl, n-Heptyl, n-Octyl, n-Nonyl, n-Decyl,-Hexyl; und insbesondere Wasserstoff oder Methyl; Phenyl.

Die übrigen Variablen sind wie oben stehend definiert.

Beispielhaft ausgewählte Verbindungen der Formel III sind (Meth)Acrylamide wie Acrylamid, N-Methylacrylamid, N,N-Dimethylacrylamid, N-Ethylacrylamid, N-Propylacryl-amid, N-tert.-Butylacrylamid, N-tert.-Octylacrylamid, N-Undecylacrylamid oder die entsprechenden Methacrylamide.

Beispielhaft ausgewählte Verbindungen der Formel IV a sind N-Vinylcarbonsäureamide wie N-Vinylformamid, N-Vinyl-N-methylfomamid, N-Vinylacetamid oder N-Vinyl-N-methylacetamid; beispielhaft ausgewählte Vertreter für Verbindungen der Formel IV b sind N-Vinylpyrrolidon, N-Vinyl-4-piperidon und N-Vinylepsilon-caprolactam.

Beispielhaft ausgewählte Verbindungen der Formel VI sind (Meth)acrylsäureester und -amide wie N,N-Dialkylaminoalkyl(meth)acrylate oder N,N-Dialkylaminoalkyl(meth)-acrylamide; Beispiele sind N,N-Dimethylaminoethylacrylat, N,N-Dimethylaminoethylmethacrylat, N,N-Diethylaminoethylacrylat, N,N-Diethylaminoethylmethacrylat, N,N-Dimethylaminopropylacrylat, N,N-Dimethylaminopropylmethacrylat, N,N-Diethylamino-propylacrylat, N,N-Diethylaminopropylmethacrylat, 2-(N,N-Dimethylamino)ethylacrylamid, 2-(N,N-Dimethylamino)ethylmethacrylamid, 2-(N,N-Diethylamino)ethylacrylamid, 2-(N,N-Diethylamino)ethylmethacrylamid, 3-(N,N-Dimethylamino)propyl-acrylamid und 3-(N,N-Dimethylarnino)propylmethacrylamid.

Beispielhaft ausgewählte Verbindungen der Formel VII sind Vinylacetat, Vinylpropionat, Vinylbutyrat, Vinyl-2-ethylhexanoat oder Vinyllaurat.

Bevorzugt werden als Monomere (D) Acrylester eingesetzt.

Ganz besonders bevorzugt wird als Monomer (D) eingesetzt: Methylacrylat, Methylmethacrylat, Acrylamid, Vinyl-n-butylether, Vinyl-iso-butylether, N-Vinylformamid, N-Vinylpyrrolidon, 1-Vinylimidazol, 4-Vinylpyridin, Vinylphosphonsäure, Vinylsulfonsäure.

In einer Ausführungsform der vorliegenden Erfindung werden die Monomere (A) bis (D) wie folgt einpolymerisiert:
5 bis 30 Gew.-% Monomer (A),
8 bis 50 Gew.-% Monomer (B),
40 bis 75 Gew.-% Monomer (C),
0 bis 20 Gew.-% Monomer (D),
wobei die Monomere wie oben stehend definiert sind.

Die Herstellung der beschriebenen Polymerisate kann wie folgt durchgeführt werden. Es ist möglich, die Monomere (A), (B), (C) und gegebenenfalls (D) durch Lösungspolymerisation, Fällungspolymerisation oder vorzugsweise lösemittelfrei durch Massepolymerisation miteinander zu copolymerisieren. Dabei können (A), (B), (C) und gegebenenfalls (D) in Form von statistischen Copolymeren oder als Blockcopolymere copolymerisieren.

Druck- und Temperaturbedingungen für eine Copolymerisation von (A), (B) (C) und gegebenenfalls (D) sind im Allgemeinen unkritisch. Die Temperaturen liegen beispielsweise im Bereich von 60 bis 200°C, bevorzugt 90 bis 160°C, der Druck liegt beispielsweise im Bereich von 1 bis 10 bar, bevorzugt 1 bis 3 bar.

Als Reaktionszeiten kommen beispielsweise 0,5 Stunden bis 12 Stunden in Frage, obwohl auch kürzere und längere Reaktionszeiten denkbar sind.

Als Lösemittel kommen vorzugsweise solche Lösemittel in Frage, die als inert gegenüber Anhydriden, die von Dicarbonsäuren mit 3 bis 8 C-Atomen abgeleitet sind, gelten, insbesondere Aceton, Tetrahydrofuran oder 1,4-Dioxan. Als Fällungsmittel eignen sich aromatische und aliphatische Kohlenwasserstoffe, beispielsweise Toluol, ortho-Xylol, meta-Xylol, para-Xylol, Ethylbenzol oder Gemische von einem oder mehreren der vorstehend genannten aromatischen Kohlenwasserstoffe, n-Hexan, Petrolether oder Isododekan. Auch Mischungen von aromatischen und aliphatischen Kohlenwasserstoffen sind geeignet. Außerdem kann auch Wasser als Lösungsmittel eingesetzt werden.

Vorzugsweise polymerisiert man in Form einer Massepolymerisation ohne den Zusatz von Lösungsmitteln.

Wählt man R6 = Wasserstoff, so kann es vorteilhaft sein, gewisse Mengen, beispielsweise 1 bis 30 Gew.-%, bezogen auf die Masse aller Monomere, an Wasser zuzusetzen und so die Bildung vernetzter Copolymerisate zu verhindern.

Man kann Regler einsetzen, beispielsweise Mercaptoethanol oder n-Dodecylmercaptan. Geeignete Mengen sind beispielsweise 0,1 bis 6 Gew.-%, bezogen auf die Masse aller Monomeren.

Die Copolymerisation startet man vorteilhaft durch Initiatoren, beispielsweise Peroxide oder Hydroperoxide. Als Peroxide bzw. Hydroperoxide seien Di-tert.-butylperoxid, tert.-Butylperoctoat, tert.-Butylperpivalat, tert.-Butylper-2-ethylhexanoat, tert.-Butylpermaleinat, tert.-Butylperisobutyrat, Benzoylperoxid, Diacetylperoxid, Succinylperoxid, p-Chlorbenzoylperoxid, Dicyclohexylperoxiddicarbonat, beispielhaft genannt. Auch der Einsatz von Redoxinitiatoren ist geeignet, außerdem Azoverbindungen wie 2,2'-Azo-bis(isobutyronitril), 2,2'-Azobis(2-methylpropion-amidin)dihydrochlorid und 2,2'-Azo-bis(4-methoxy-2,4-dimethylvaleronitril). Im allgemeinen werden diese Initiatoren in Mengen von 0,1 bis 20 Gew.-%, vorzugsweise 0,2 bis 15 Gew.-%, berechnet auf die Masse aller Monomeren, eingesetzt.

Durch die oben beschriebene Copolymerisation erhält man Polymerisate. Die anfallenden Polymerisate können Verbindung der allgemeinen Formel I enthalten, und man kann sie einer Reinigung nach konventionellen Methoden unterziehen, beispielsweise Umfällen oder extraktive Entfernung nichtumgesetzter Monomere. Man kann aber die Reinigung unterlassen und die oben beschriebenen Copolymerisate im Gemisch mit Verbindung I einsetzen. Wenn ein Lösemittel oder Fällungsmittel eingesetzt wurde, so ist es möglich, dieses nach beendeter Copolymerisation zu entfernen, beispielsweise durch Abdestillieren.

Die Polydispersität der oben beschriebenen Polymerisate liegt im Allgemeinen zwischen 2 und 10, bevorzugt bis 7, kann aber höhere Werte annehmen.

Die K-Werte der oben beschriebenen Polymerisate betragen 6 bis 100, vorzugsweise 10 bis 60 (gemessen nach H. Fikentscher bei 25 °C in beispielsweise Wasser oder Tetrahydrofuran und einer Polymerkonzentration von 1 Gew.-%).

Die Herstellung von Monomeren der allgemeinen Formel I ist an sich bekannt.

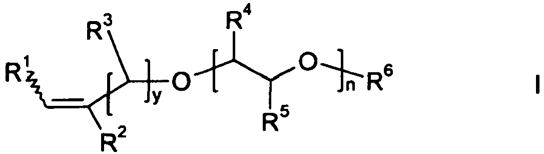

Im Folgenden wird die Erfindung unter Bezugnahme auf die Beispiele näher beschrieben.

### Beispiele:

Verschiedene schlecht wasserlösliche Substanzen wurden eingesetzt, um die Eignung der erfindungsgemäßen Polymere als Lösungsvermittler zu testen.

**Tabelle 1**

| Substanz | Löslichkeit in destilliertem Wasser (mg/l) |
|---|---|
| Pyren | 0.13 |
| Uvinul T150® (Octyltriazon) | 0.007 |
| Cl solvent Red® (Farbstoff) | < 0.001 |
| Cinidon-Ethyl (Herbizid) | 0.057 |
| Piroxicam (Schmerzmittel) | 23 |
| Pyraclostrobin (Fungizid) | 2,4 |
| Metconazol (Fungizid) | 15 |

Um die Eignung der Polymere als Lösungsvermittler zu testen wurden die folgenden Maßnahmen durchgeführt:

### Verfahren 1: Feste Lösung

0,5 g eines Polymers und 0,1 g einer ausgewählten Substanz wurden in DMF (ca. 20 ml) gelöst. Das Gemisch wurde gerührt und DMF wurde vollständig evaporiert, so daß eine Flüssigdispersion der organischen Moleküle in dem Polymer erhalten wurde. Eine gepufferte wäßrige Lösung (100 ml, pH 6,8) wurde hinzugeführt und das Gemisch für 24 Stunden gerührt. Es werden stabile Lösungen bzw. Dispersionen erhalten, d.h. auch nach ca. einer Woche war keine Sedimentation zu beobachten. Nach der Filtration wurde die Lösung mittels HPLC analysiert (Detektor UV) und die Konzentration der Substanzen bestimmt.

### Verfahren 2: Schmelzemulgierung

10 g Wirkstoffschmelze wurden in 90 g wäßrige 65°C warme Polymerlösung enthaltend 30 g Polymer eingerührt. Je nach Viskosität von Polymerlösung und Wirkstoff wurden zur Solubilisierung ein Magnetrührer oder ein Ultraturrax verwendet. Die erforderliche Zeit bis zum Solubilisierungsgleichgewicht ist abhängig von Polymer und Wirkstoff und kann Sekunden bis Stunden dauern. Es werden stabile Lösungen bzw. Dispersionen erhalten, d.h. auch nach ca. einer Woche war keine Sedimentation zu beobachten.

### Verfahren 3: Phaseninversion

10 g des flüssigen oder festen Wirkstoffs und 30 g Polymer wurden in einem organischen Lösemittel gelöst, welches einen Siedepunkt unter 100°C hat. Dann wurde unter Rühren Wasser zugegeben und anschließend das organische Lösemittel am Rotationsverdampfer entfernt. Es wurde soviel Wasser zugegeben, daß die resultierende wäßrige Formulierung 10%ig an Wirkstoff und 30%ig an Polymer war. Es werden stabile Lösungen bzw. Dispersionen erhalten.

### Beispiele für die Herstellung der Polymere

### Herstellungsbeispiel 1: Herstellung eines Terpolymers (Pluriol A10R/Acrylsäure/Styrol) (1:1:1)

373,5 g (0,75 mol) Pluriol A010R und 78 g (0,77 mol) Essigsäureanhydrid wurden unter Rühren auf 150°C erhitzt und eine Stunde bei 150°C gehalten. Anschließend wurden innerhalb von 5 Stunden 54 g (0,75 mol) Acrylsäure, 78,0 g (0,75 mol) Styrol und 10,1 g Di-tert.-Butylperoxid zudosiert. Anschließend wurde eine halbe Stunde bei 150°C nacherhitzt.
K-Wert = 17 (1 % in Wasser)

### Herstellungsbeispiel 2: Herstellung eines Terpolymers (Pluriol A10R/Acrylsäure/Styrol) (1:1:3)

373,5 g (0,75 mol) Pluriol A010R wurden in einem 2-I-Kessel vorgelegt und im schwachen Stickstoffstrom auf 150°C erhitzt. Nach Erreichen dieser Temperatur wurden innerhalb von 5 Stunden 54 g (0,75 mol) Acrylsäure, 234,0 g Styrol und 13,23 g Di-tert.-Butylperoxid zudosiert. Anschließend wurde der Ansatz eine halbe Stunde bei 150°C gehalten.
K-Wert (1 % in Wasser) = 23
GPC in N,N-Dimethylacetamid (PMMA-Standard): Mn = 3.100, Mw = 63.000

### Formulierungsbeispiele

### Formulierungsbeispiel 1:

Solubilisationsergebnisse mit Polymer aus Herstellungsbeispiel 1 nach Verfahren 1:

**Tabelle 2**

| Wirkstoff | Löslichkeit in destilliertem Wasser [mg/l] | Löslichkeit in Gegenwart von Polymer [mg/l] |
|---|---|---|
| Uvinul T 150® | 0.007 | 2,3 |
| Cinidon -Ethyl | 0.057 | 0,5 |
| Pyren | 0.13 | 7 |
| Piroxicam | 23 | 92 |

### Formulierungsbeispiel 2:

Solubilisierungsergebnisse mit Polymer aus Herstellungsbeispiel 2 nach Verfahren 1:

**Tabelle 3**

| Wirkstoff | Löslichkeit in destilliertem Wasser [mg/l] | in Gegenwart von Polymer [mg/l] |
|---|---|---|
| Uvinul T 150® | 0.007 | 79 |
| Cinidon-Eethyl | J.057 | 57 |
| Pyren | 0.13 | 63 |
| Cl solvent Red® | < 0.001 | 0,2 |
| Piroxicam | 23 | 167 |

### Formulierungsbeispiel 3:

### Solubilisierung von Pyraclostrobin mit Polymer aus Herstellbeispiel 2 nach Verfahren 3

10 g des Fungizids Pyraclostrobin wurden zusammen mit 30 g Polymer aus Beispiel 2 in 100 g THF gelöst. Dann wurde unter Rühren (Magnetrührer) soviel Wasser zugegeben, daß nach Entfernen des organischen Lösemittels am Rotationsverdampfer die resultierende wäßrige Formulierung 10% Wirkstoff und 30% Polymer enthielt.

Der Wirkstoff Pyraclostrobin ist bei 70°C eine gut fließende Schmelze (Viskosität 2200 mPas) und kann auch nach Verfahren 2 formuliert werden. Die Formulierung ist in beiden Fällen homogen, mindestens mehrere Monate sedimentationsstabil und läßt sich mit Wasser (geprüft mit VE-Wasser und 10°dH) sedimentations- und kristallisationsstabil verdünnen.

Die Polymer-Wirkstoff-Partikel sind sphärisch und haben einen Durchmesser von ca. 30 nm. Im Elektronenmikroskop (TEM) können keine Wirkstoffkristalle nachgewiesen werden.

Die nanopartikuläre Fungizidformulierung, die nur Wasser, Polymer und Wirkstoff enthielt, wurde mit einer kommerziellen Formulierungen des Wirkstoffs Pyraclostrobin bezüglich der biologischen Wirkung im Gewächshaus verglichen.

Dazu wurden die Wirkstoffformulierungen mit Leitungswasser auf die gewünschte Wirkstoffkonzentrationen (zwischen 4 und 250 ppm) verdünnt. Die Applikation auf Tomatenpflanzen erfolgte in einer Spritzkabine mit 25 ml Volumen, welches einer praxisüblichen Wassermenge von ca. 500 I/ha entspricht. Der Standardpilz (Phytophthora infestans) wurde 7 Tage nach Behandlung inokuliert. Die Versuchspflanzen wurden im Gewächshaus bei 18-20°C und 90% relativer Luftfeuchte aufgestellt. Die Bonitur erfolgte 5 Tage nach Inokulation durch Bestimmung des prozentualen Befalls der Blätter.

Die Ergebnisse der biologischen Prüfung sind in der folgenden Tabelle zusammengefasst (Tabelle 4).

**Tabelle 4: Phytophthora infestans - Befall [%] an Tomaten nach fünf Tagen als Funktion des Wirkstoffgehalts**

| | Befall [%] | Befall [%] |
|---|---|---|
| Aufwandmenge [ppm] | Formulierung Beispiel 3 | Verkaufsprodukt¹ |
| 250 | 0 | 0 |
| 63 | 0 | 0 |
| 16 | 0 | 0 |
| 4 | 3 | 4 |

| | | |
|---|---|---|
| 1) EC-Formulierung: 23,5 Gew.-% Pyraclostrobin, 4,7 Gew.-% anionisches Netzmittel und 4,7 Gew.-% nichtionisches Netzmittel in 67,1 Gew.-% eines aromatischen Lösungsmittels | | |

### Formulierungsbeispiel 4:

### Solubilisierung von Pyraclostrobin aus Herstellbeispiel 2 nach Verfahren 2

Pyraclostrobin ist bei 70°C eine gut fließende Schmelze (Viskosität 2200 mPas) und wurde auch nach der allgemeinen Vorschrift 2 bei einer Temperatur von 70°C formuliert. Die erhaltene Wirkstoffzusammensetzung war homogen, nahezu visuell transparent, mindestens mehrere Monate sedimentationsstabil und ließ sich mit Wasser (sowohl mit vollentsalztem Wasser und Wasser 10°dH) verdünnen, ohne dass eine Sedimendation oder eine Kristallisation des Wirkstoffs auftrat. Je nach Wirkstoff-Polymer-Verhältnis (zwischen 1:2,5 und 1:1) wurden in der dynamischen Lichtstreuung mittlere Durchmesser zwischen 30 und 65 nm gemessen. Im Transmissionelektronenmikroskop konnten keine Wirkstoffpartikel nachgewiesen werden.

Die nanopartikuläre Fungizidformulierung, die nur Wasser, Polymer und Wirkstoff enthielt, wurde mit einer kommerziellen Formulierungen des Wirkstoffs Pyraclostrobin bezüglich der biologischen Wirkung im Gewächshaus verglichen.

Dazu wurden die Wirkstoffformulierungen mit Leitungswasser auf die gewünschte Wirkstoffkonzentrationen (zwischen 4 und 250 ppm) verdünnt. Die Applikation auf Tomatenpflanzen erfolgte in einer Spritzkabine mit 25 ml Volumen, welches einer praxisüblichen Wassermenge von ca. 500 l/ha entspricht. Der Standardpilz (Phytophthora infestans) wurde 7 Tage nach Behandlung inokuliert. Die Versuchspflanzen wurden im Gewächshaus bei 18-20°C und 90% relativer Luftfeuchte aufgestellt. Die Bonitur erfolgte 5 Tage nach Inokulation durch Bestimmung des prozentualen Befalls der Blätter.

**Tabelle 4: Phytophthora infestans - Befall [%] an Tomaten nach fünf Tagen als Funktion des Wirkstoffgehalts**

| | Befall [%] | Befall [%] |
|---|---|---|
| Aufwandmenge [ppm] | Formulierung Beispiel 4 | Verkaufsprodukt¹ |
| 250 | 0 | 0 |
| 63 | 0 | 0 |
| 16 | 0 | 0 |
| 4 | 6 | 4 |

| | | |
|---|---|---|
| 1) EC-Formulierung: 23,5 Gew.-% Pyraclostrobin, 4,7 Gew.-% anionisches Netzmittel und 4,7 Gew.-% nichtionisches Netzmittel in 67,1 Gew.-% eines aromatischen Lösungsmittels | | |

## Patentansprüche

1. Wässrige Wirkstoffzusammensetzung enthaltend Wasser,
amphiphile Polyethergruppen enthaltende Polymere als Lösungsvermittler und
als Wirk- und/oder Effektstoff Herbizide, Fungizide, Nematozide, Akarizide, Insektizide, Wirkstoffe, die das Pflanzenwachstum regulieren und pharmazeutische Wirkstoffe,
wobei die Polymere erhältlich sind durch Copolymerisation von
(A) 5 bis 30 Gew.-% (Meth)acrylsäure oder Maleinsäureanhydrid,
(B) 5 bis 80 Gew.-% Styrol,
(C) 10 bis 90 Gew.-% mindestens eines alkoxylierten ungesättigten Ethers der allgemeinen Formel I 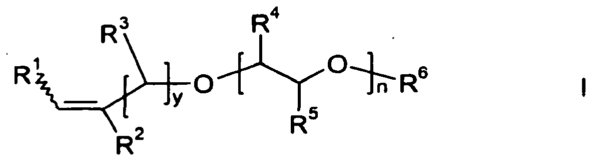
(D) 0 bis 20 Gew.-% weiteren monoethylenisch ungesättigten Monomeren,
wobei in Formel I die Variablen wie folgt definiert sind:
R¹ und R³ Wasserstoff;
R² gewählt aus Wasserstoff oder Methyl;
R⁴, R⁵ jeweils Wasserstoff;
R⁶ gewählt aus Wasserstoff oder C₁-C₄-Alkyl,
n eine ganze Zahl von 3 bis 100,
y 1.

2. Wässrige Wirkstoffzusammensetzung nach Anspruch 1, **dadurch gekennzeichnet, dass** in Formel I der Rest R⁶ Wasserstoff ist.

3. Wässrige Wirkstoffzusammensetzung nach einem der Ansprüche 1 oder 2, **dadurch gekennzeichnet, dass** als Wirk- und/oder Effektstoff Herbizide, Fungizide, Nematozide, Akarizide, Insektizide, Wirkstoffe, die das Pflanzenwachstum regulieren, enthalten sind.

4. Wässrige Wirkstoffzusammensetzung nach einem der Ansprüche 1 bis 3, **dadurch gekennzeichnet, dass** der Anteil an organischen Lösungsmitteln nicht mehr als 10 Gew.-% beträgt.

5. Wässrige Wirkstoffzusammensetzung nach einem der Ansprüche 1 bis 4, **dadurch gekennzeichnet, dass** (D) gewählt wird aus ethylenisch ungesättigten C₃-C₈-Carbonsäurederivaten der allgemeinen Formel II 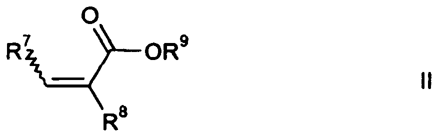 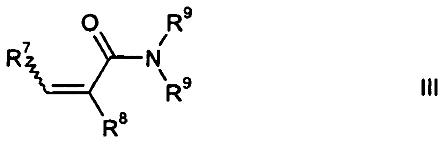 Acrylamiden der Formel III,
nicht-cyclischen Amiden der allgemeinen Formel IV a oder cyclischen Amiden der allgemeinen Formel IV b 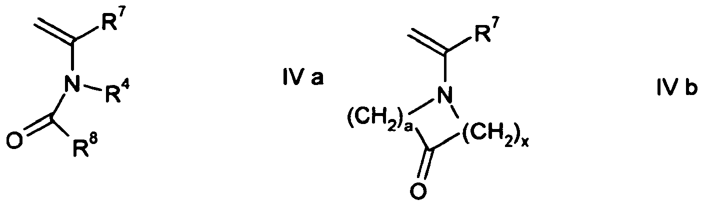 C₁-C₂₀-Alkyl-Vinylethern,
N-Vinyl-Derivaten von stickstoffhaltigen aromatischen Verbindungen, alkoxylierten ungesättigten Estern der allgemeinen Formel V, 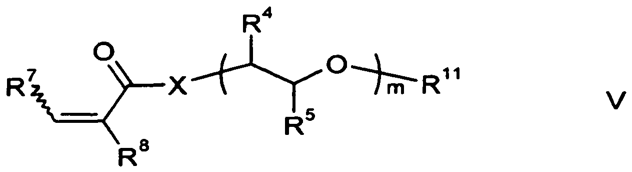 Estern oder Amiden der allgemeinen Formel VI 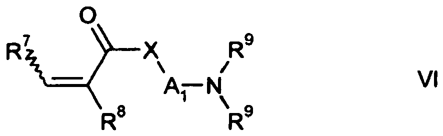 ungesättigten Estern der allgemeinen Formel VII 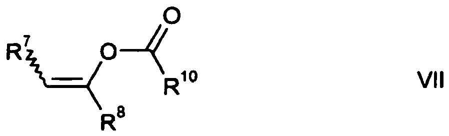 Sulfonat-, Phosphat- oder Phosphonat-Gruppen-haltigen Monomeren, wobei in den allgemeinen Formeln II bis VII die Variablen wie folgt definiert sind:
R⁷, R⁸ gleich oder verschieden und gewählt aus Wasserstoff und C₁-C₁₀-Alkyl,
R⁹ gleich oder verschieden und gewählt aus Wasserstoff oder C₁-C₂₂-Alkyl mit der Maßgabe, dass in Formel II R⁹ nicht für Wasserstoff steht.
A₁ C₂-C₆-Alkylen, verzweigt oder unverzweigt;
x eine ganze Zahl im Bereich von 2 bis 100,
a eine ganze Zahl im Bereich von 0 bis 6,
m eine ganze Zahl im Bereich von 2 bis 200,
R¹⁰, R¹¹ gleich oder verschieden und gewählt aus Wasserstoff und C₁-C₁₀-Alkyl,
X Sauerstoff oder N-R¹²
R¹² gewählt aus Wasserstoff, C₁-C₁₀-Alkyl, Phenyl,
und die übrigen Variablen wie oben stehend definiert sind, mit der Maßgabe, dass in der Formel II R⁹ nicht für Wasserstoff steht.

6. Wässrige Wirkstoffzusammensetzung nach einem der Ansprüche 1 bis 5, **dadurch gekennzeichnet, dass** (A) (Meth)Acrylsäure ist.

7. Wässrige Wirkstoffzusammensetzung nach einem der Ansprüche 1 bis 6, **dadurch gekennzeichnet, dass** in Formel I der Index n 3-40 ist.

8. Wässrige Wirkstoffzusammensetzung nach einem der Ansprüche 1 bis 7, **dadurch gekennzeichnet, dass**
5 bis 30 Gew.-% Monomer (A),
8 bis 50 Gew.-% Monomer (B),
40 bis 75 Gew.-% Monomer (C),
0 bis 20 Gew.-% Monomer (D),
einpolymerisiert werden.

## Claims

1. An aqueous active substance composition comprising water,
polymers comprising amphiphilic polyether groups, as solubilizers, and
herbicides, fungicides, nematicides, acaricides, insecticides, active substances which regulate plant growth and pharmaceutical active substances as active substance and/or effective substance, where the polymers are obtainable by copolymerization of
(A) 5 to 30% by weight of (meth)acrylic acid or maleic anhydride,
(B) 5 to 80% by weight of styrene,
(C) 10 to 90% by weight of at least one alkoxylated unsaturated ether of the general formula I 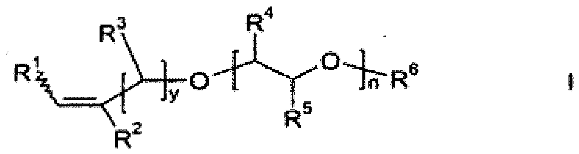
(D) 0 to 20% by weight of further monoethylenically unsaturated monomers, where, in formula I, the variables are defined as follows:
R¹ and R³ are hydrogen;
R² is selected from among hydrogen or methyl;
R⁴, R⁵ are in each case hydrogen;
R⁶ is selected from among hydrogen or C₁-C₄-alkyl,
n is an integer from 3 to 100,
y is 1.

2. The aqueous active substance composition according to claim 1, wherein, in formula I, the radical R⁶ is hydrogen.

3. The aqueous active substance composition according to one of claims 1 or 2, wherein herbicides, fungicides, nematicides, acaricides, insecticides, active substances which regulate plant growth are present as active substance and/or effective substance.

4. The aqueous active substance composition according to any of claims 1 to 3, wherein the organic solvent content amounts to not more than 10% by weight.

5. The aqueous active substance composition according to any of claims 1 to 4, wherein (D) is selected from among
ethylenically unsaturated C₃-C₈-carboxylic acid derivatives of the general formula II 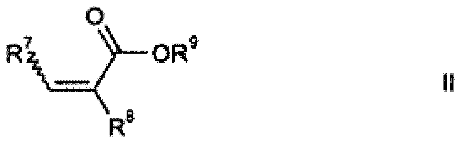 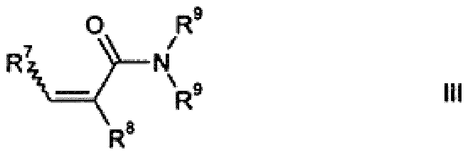 acrylamides of the formula III,
non-cyclic amides of the general formula IV a or cyclic amides of the general formula IV b 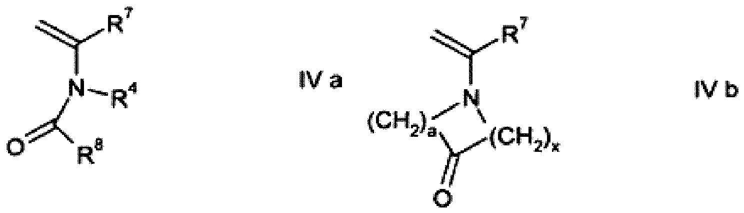 C₁-C₂₀-alkyl vinyl ethers,
N-vinyl derivatives of nitrogen-containing aromatic compounds, alkoxylated unsaturated esters of the general formula V, 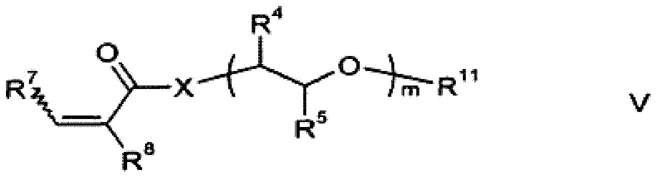 esters or amides of the general formula VI 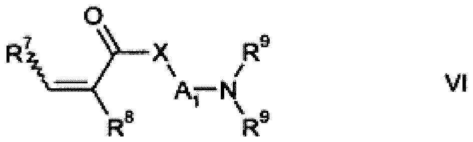 unsaturated esters of the general formula VII 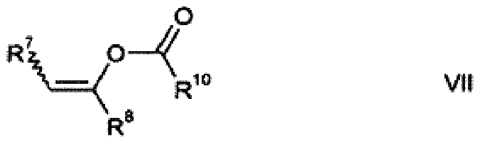 sulfonate-, phosphate- or phosphonate-group-containing monomers,
where in the general formulae II to VII the variables are defined as follows:
R⁷, R⁸ are identical or different and selected from among hydrogen and C₁-C₁₀-alkyl,
R⁹ are identical or different and selected from among hydrogen or C₁-C₂₂-alkyl, with the proviso that, in formula II, R⁹ is other than hydrogen,
A₁ is branched or unbranched C₂-C₆-alkylene;
x is an integer in the range of from 2 to 100,
a is an integer in the range of from 0 to 6,
m is an integer in the range of from 2 to 200,
R¹⁰, R¹¹ are identical or different and are selected from among hydrogen and C₁-C₁₀-alkyl,
X is oxygen or N-R¹²,
R¹² is selected from among hydrogen, C₁-C₁₀-alkyl, phenyl,
and the remaining variables are defined as hereinabove, with the proviso that, in formula II, R⁹ is other than hydrogen.

6. The aqueous active substance composition according to any of claims 1 to 5, wherein (A) is (meth) acrylic acid.

7. The aqueous active substance composition according to any of claims 1 to 6, wherein, in formula I, the index n is 3-40.

8. The aqueous active substance composition according to any of claims 1 to 7, wherein
5 to 30% by weight of monomer (A),
8 to 50% by weight of monomer (B),
40 to 75% by weight of monomer (C),
0 to 20% by weight of monomer (D),
are incorporated into the polymer.

## Revendications

1. Composition aqueuse de matières actives, contenant : de l'eau,
des polymères contenant des groupes polyéther amphiphiles, servant de tiers-solvants, et
en tant que matières actives et/ou substances présentant des effets, des herbicides, des fongicides, des nématicides, des acaricides, des insecticides, des matières actives qui régulent la croissance des végétaux, et des substances actives pharmaceutiques, les polymères pouvant être obtenus par polymérisation de :
(A) 5 à 30 % en poids d'acide (méth)acrylique ou d'anhydride maléique,
(B) 5 à 80 % en poids de styrène,
(C) 10 à 90 % en poids d'au moins un éther insaturé alcoxylé de formule générale I 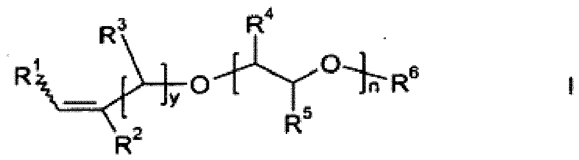
(D) 0 à 20 % en poids d'autres monomères à insaturation monoéthylénique,
où, dans la formule I, les variables sont définies comme suit :
R¹ et R³ sont des atomes d'hydrogène ;
R² est choisi parmi l'atome d'hydrogène ou le groupe méthyle ;
R⁴ et R⁵ représentent chacun un atome d'hydrogène ;
R⁸ est choisi parmi l'atome d'hydrogène ou les groupes alkyle en C₁-C₄,
n est un nombre entier de 3 à 100,
y vaut 1.

2. Composition aqueuse de matières actives selon la revendication 1, **caractérisée en ce que**, dans la formule I, le radical R⁸ est un atome d'hydrogène.

3. Composition aqueuse de matières actives selon l'une des revendications 1 ou 2, **caractérisée en ce qu'**elle contient en tant que matière active et/ou substance présentant des effets des herbicides, des fongicides, des nématicides, des acaricides, des insecticides, des matières actives qui régulent la croissance des végétaux.

4. Composition aqueuse de matières actives selon l'une des revendications 1 à 3, **caractérisée en ce que** la proportion des solvants organiques n'est pas supérieure à 10 % en poids.

5. Composition aqueuse de matières actives selon l'une des revendications 1 à 4, **caractérisée en ce que** (D) est choisi parmi
- les dérivés d'acides carboxyliques en C₃-C₈ à insaturation éthylénique de formule générale II 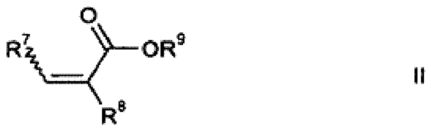 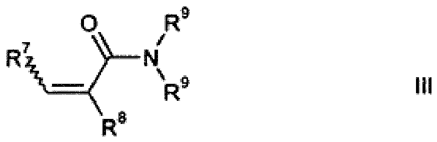
- les acrylamides de formule III,
- les amides non cycliques de formule générale IVa ou les amides cycliques de formule générale IVb 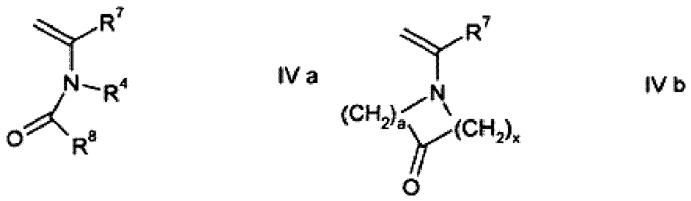
- les (alkyle en C₁-C₂₀) vinyléthers,
- les dérivés N-vinyliques de composés aromatiques azotés,
- les esters insaturés alcoxylés de formule générale V, 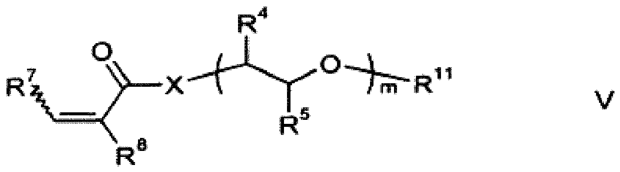
- les esters ou amides de formule générale VI, 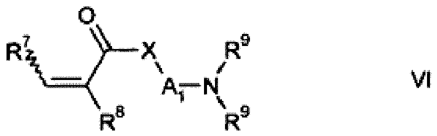
- les esters insaturés de formule générale VII 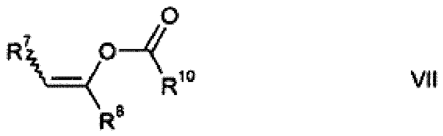
- les monomères contenant des groupes sulfonate, phosphate ou phosphonate,
où, dans les formules générales II à VII, les variables sont définies comme suit :
R⁷, R⁸ sont identiques ou différents et sont choisis parmi l'atome d'hydrogène et les groupes alkyle en C₁-C₁₀,
les radicaux R⁹ sont identiques ou différents et sont choisis parmi l'atome d'hydrogène ou les groupes alkyle en C₁-C₂₂, à la condition que, dans la formule II, R⁹ ne soit pas un atome d'hydrogène,
A₁ est un groupe alkylène en C₂-C₆ à chaîne droite ou ramifiée ;
x est un nombre entier compris dans la plage de 2 à 100,
a est un nombre entier compris dans la plage de 0 à 6,
m est un nombre entier compris dans la plage de 2 à 200,
R¹⁰, R¹¹ sont identiques ou différents et sont choisis parmi l'atome d'hydrogène et les groupes alkyle en C₁-C₁₀,
X est un atome d'oxygène ou N-R¹²,
R¹² est choisi parmi l'atome d'hydrogène, les groupes alkyle en C₁-C₁₀, phényle,
et les autres variables sont définies comme mentionné ci-dessus, à la condition que, dans la formule II, R⁹ ne soit pas un atome d'hydrogène.

6. Composition aqueuse de matières actives selon l'une des revendications 1 à 5, **caractérisée en ce que** (A) est l'acide (méth)acrylique.

7. Composition aqueuse de matières actives selon l'une des revendications 1 à 6, **caractérisée en ce que**, dans la formule I, l'indice n vaut 3-40.

8. Composition aqueuse de matières actives selon l'une des revendications 1 à 7, **caractérisée en ce qu'**elle contient, polymérisés :
5 à 30 % en poids du monomère (A),
8 à 50 % en poids du monomère (B),
40 à 75 % en poids du monomère (c),
0 à 20 % en poids du monomère (D).
